(19) Europäisches Patentamt
European Patent Office
Office européen des brevets

(11) EP 1 167 360 A1

(12) EUROPEAN PATENT APPLICATION
published in accordance with Art. 158(3) EPC

(43) Date of publication:
02.01.2002 Bulletin 2002/01

(21) Application number: 01901398.6

(22) Date of filing: 17.01.2001

(51) Int Cl.[7]: **C07D 235/28**, C07D 235/16, C07D 471/04, A61K 31/4184, A61K 31/437, A61P 43/00, A61P 29/00, A61P 37/08, A61P 11/00, A61P 9/00, A61P 3/14

(86) International application number:
PCT/JP01/00272

(87) International publication number:
WO 01/53272 (26.07.2001 Gazette 2001/30)

(84) Designated Contracting States:
AT BE CH CY DE DK ES FI FR GB GR IE IT LI LU MC NL PT SE TR
Designated Extension States:
AL LT LV MK RO SI

(30) Priority: 17.01.2000 JP 2000007532
19.01.2000 JP 2000010406

(71) Applicant: TEIJIN LIMITED
Osaka-shi Osaka 541-0054 (JP)

(72) Inventors:
- TSUCHIYA, Naoki, c/o Teijin Ltd, Tokyo Res.Center Hino-shi, Tokyo 191-0065 (JP)
- MATSUMOTO, Yoshiyuki, c/o Teijin Ltd Hino-shi, Tokyo 191-0065 (JP)
- SAITOU, Hiroshi, c/o Teijin Ltd, Tokyo Res. Center Hino-shi, Tokyo 191-0065 (JP)
- MIZUNO, Tsuyoshi, c/o Teijin Ltd,Tokyo Res. Center Hino-shi, Tokyo 191-0065 (JP)

(74) Representative: Hallybone, Huw George et al
Carpmaels and Ransford, 43 Bloomsbury Square
London WC1A 2RA (GB)

(54) **HUMAN CHYMASE INHIBITORS**

(57) The present invention provides a benzimidazole derivative or its pharmaceutically permissible salt expressed by the following formula (1). Further, the present invention provides a human chymase activity inhibitor containing the substance as an active ingredient.

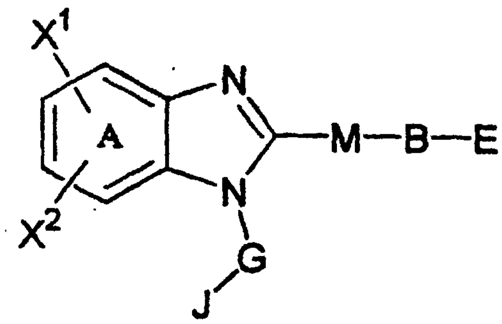

(1)

(the ring marked with A expresses a pyridine ring or a benzene ring; $X^1$ and $X^2$ are each a hydrogen atom, a halogen atom, a trihalomethyl group, a cyano group, a substituted or unsubstituted alkyl group, a substituted or unsubstituted alkoxy group, or the like; B is a substituted or unsubstituted alkylene group, or the like; E is $-COOR^4$ or the like; G is a substituted or unsubstituted alkylene group; J is a substituted or unsubstituted alkyl group, or a substituted or un-

EP 1 167 360 A1

substituted aryl group; and M is a sulfur atom, sulfoxide, sulfone or the like).

## Description

### Technical Field

The present invention relates to a human chymase inhibitor useful as a medicine. More specifically, the invention relates to a new benzimidazole derivative useful as an active ingredient of an inhibitor against human chymase activity.

### Background Art

A chymase exists in a granule of a mast cell, and it is one of neutral proteinases and deeply relates to the various vital reactions to which the mast cell relates. For example, there are many reports such as the acceleration of degranulation from a mast cell, the activation of interleukin-1 β (IL-1 β), the activation of matrix protease, the degradation of fibronectin or IV-type collagen, the acceleration of release of transforming growth factor- β (TGF- β), the activation of substance-P or vathoactive intestinal polypeptide (VIP), the conversion of angiotensin (Ang) I to Ang II, and the conversion of endothelin. From the above mentioned things, the inhibitor against the activity of said chymase is considered to be promising as a compound applicable to a preventing agent and/or treating agent for a respiratory disease such as bronchial asthma; an inflammatory/allergy disease such as allergic rhinitis, atopic dermatitis or urticaria; a cardiovascular disease such as sclerosing vascular lesion, intravascular stenosis, peripheral circulatory disorder, kidney failure or cardiac failure; a bone/cartridge metabolic disease such as rheumatism or osteoarthritis; or the like.

Heretoforth, as chymase activity inhibitors, a triazine derivative (JP-A 8-208654) (JP-A means Japanese Unexamined Patent Publication), a hydantoin derivative (JP-A 9-31061), an imidazolidine derivative (International Patent Publication WO96/04248), a quinazoline derivative (International Patent Publication WO97/11941), a heterocyclic amido derivative (International Patent Publication WO96/33974) and the like have been known.

However, regarding the triazine derivative, the inhibiting activity was studied by using a rat chymase, and the inhibiting activity against a human chymase is unclear. Regarding the hydantoin derivative, the inhibiting activity against a human chymase was investigated, but the activity is overall weak and insufficient. Regarding the imidazolidine derivative, there is the high possibility that it is an irreversible inhibitor (suicide substrate) of a chymase judging from its chemical structure. Further, all the structures of the abovementioned substances are widely different from the structures of benzimidazole derivatives of the present description.

On the other hand, as the relevant technology of the benzimidazole derivative of the present invention, there are benzimidazole derivatives described in United State Patents 5,021,443, 5,124,336 and 5,128,339. However, there is neither description nor suggestion regarding compounds having a cyano group, $-CH_2NH_2$, $-CH=NR^1$ or $-CONR^1R^2$ ($R^1$ and $R^2$ are each a hydrogen atom or a $C_{1-4}$ alkyl group) at the position of 4, 5, 6 or 7 in a benzimidazole skeleton. Further, said patent publications describe only antagonists against a thromboxane receptor, and there is neither description nor suggestion on an inhibiting activity against a human chymase.

### Disclosure of the Invention

It is an object of the present invention to provide an inhibitor against human chymase activity containing a benzimidazole derivative as the active ingredient and useful for clinical applications.

Further, the object of the present invention is to provide a new benzimidazole derivative or its pharmaceutically permissible salt to be the active ingredient.

The inventors of the present invention repeatedly pursued zealous studies in order to achieve the abovementioned objects, and found that the imidazole derivative expressed by the following formula (1) or its salt has an inhibiting activity selectively against a human chymase in spite that it is completely different in its structure from a serine proteinase inhibitor, which has been heretofore reported.

That is, the present invention provides 'an inhibitor against human chymase activity containing a benzimidazole derivative expressed by the following formula (1) or its salt as the active ingredient and useful for clinical applications.

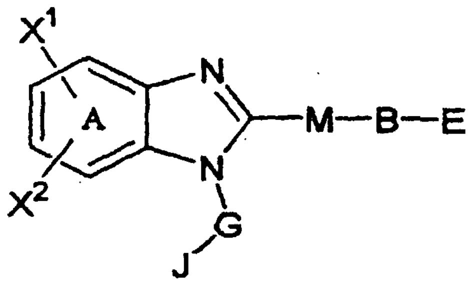

(1)

[in the formula (1), the ring marked with A expresses a pyridine ring or a benzene ring;

$X^1$ and $X^2$ are each at the same time or independently a hydrogen atom, a halogen atom, a trihalomethyl group, a hydroxyl group, a nitro group, a cyano group, $-CH_2NH_2$, $-CH=NR^1$, $-CH=NOR^1$ or $-CONR^1R^2$ (here, $R^1$ and $R^2$ are each a hydrogen atom or a $C_{1-4}$ alkyl group), $-COOR^3$ (here, $R^3$ is a hydrogen atom or a $C_{1-4}$ alkyl group), a substituted or unsubstituted $C_{1-6}$ normal, cyclic or branched alkyl group, a substituted or unsubstituted $C_{3-7}$ cycloalkyl group, a substituted or unsubstituted $C_{1-6}$ normal or branched alkoxyl group, a substituted or unsubstituted $C_{1-6}$ normal or branched alkylthio group, a substituted or unsubstituted $C_{1-6}$ normal or branched alkylsulfonyl group, or a substituted or unsubstituted $C_{1-6}$ normal or branched alkylsulfinyl group {the substituent permissible to the groups is a halogen atom, a hydroxyl group, a nitro group, a cyano group, an acyl group, a trihalomethyl group, a trihalomethoxy group, a phenyl group, an oxo group or a phenoxy group optionally substituted with one or more halogen atoms, and the substituent may substitute singly or plurally independently at arbitrary position(s)};

B is a substituted or unsubstituted $C_{1-6}$ normal, cyclic or branched alkylene group or a substituted or unsubstituted $C_{2-6}$ normal or branched alkenylene group {the substituent permissible to the groups is a halogen atom, a hydroxyl group, a nitro group, a cyano group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched acyl group, a $C_{1-6}$ normal or branched acylamino group, a trihalomethyl group, a trihalomethoxy group, a phenyl group, an oxo group or a phenoxy group optionally substituted with one or more halogen atoms, and the substituent may substitute singly or plurally independently at arbitrary position(s) of the alkylene group or an alkenylene group; between atoms, the alkylene group or alkenylene group optionally contains one or more of -O-, -S-, $-SO_2-$ or $-NR^4-$, but said atom or atomic group does not bond directly to the M, and here $R^4$ is a hydrogen atom or a $C_{1-6}$ normal or branched alkyl group};

E expresses $-COOR^4$, $-SO_3R^4$, $-CONHR^5$, $-SO_2NHR^4$, $-PO(OR^6)_2$, a tetrazol-5-yl group, a 5-oxo-1,2,4-oxadiazol-3-yl group or a 5-oxo-1,2,4-thiadiazol-3-yl group (here, $R^4$ is similarly defined as above; $R^5$ is a hydrogen atom, a cyano group, or a $C_{1-6}$ normal or branched alkyl; $R^6$ is a hydrogen atom, a $C_{1-6}$ normal or branched alkyl group, or a trifluoromethylsulfonyl group, or its pharmaceutically permissible salt);

G is a substituted or unsubstituted $C_{1-6}$ normal or branched alkylene group {between atoms, the alkylene group optionally contains one or more of -O-, -S-, $-SO_2-$ or $-NR^{4-}$, but this atom or atomic group does not bond directly to the nitrogen atom of the imidazole ring ($R^4$ is similarly defined as above), and the substituent is a halogen atom, a hydroxyl group, a nitro group, a cyano group, a $C_{1-6}$ normal or branched alkoxyl group (including the case where adjacent two groups form an acetal bonding), a trihalomethyl group, a trihalomethoxy group, a phenyl group or an oxo group};

J is a substituted or unsubstituted $C_{1-6}$ normal, cyclic or branched alkyl group, a substituted or unsubstituted $C_{4-10}$ aryl group {the substituent permissible to the groups is a halogen atom, a hydroxyl group, a nitro group, a cyano group, $-COOR^7$ (here, $R^7$ is a hydrogen atom or a $C_{1-4}$ alkyl group), a $C_{1-6}$ normal, cyclic or branched alkyl group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched alkylsulfinyl group, a $C_{1-6}$ acyl group, a $C_{1-6}$ normal or branched acylamino group, a trihalomethyl group, a trihalomethoxy group, a phenyl group, an oxo group, or a phenoxy group optionally substituted with one or more halogen atoms; the substituent may substitute singly or plurally independently at arbitrary position(s) of the alkyl group or aryl group; and the substituent is further optionally substituted with a halogen atom, a hydroxyl group, a nitro group, a cyano group, an acyl group, a trihalomethyl group, a phenyl group, an oxo group or a phenoxy group optionally substituted with a halogen atom}; and

M is a sulfur atom, a sulfinyl group, a sulfonyl group, a single bond or $-CR^8R^{9-}$ (here, $R^8$ and $R^9$ are each at the same time or independently a hydrogen atom or a $C_{1-4}$ alkyl group)].

Here, a part of the compounds expressed by the above formula (1) are new compounds, and the present invention also provides the imidazole derivatives like this or their salts.

**Best Mode for Carrying Out the Invention**

The substituents of the compound expresses by the above formula (1) of the present invention are defined as follows:

The ring marked with A expresses a pyridine ring or a benzene ring.

$X^1$ and $X^2$ are each at the same time or independently a hydrogen atom, a halogen atom, a trihalomethyl group, a hydroxyl group, a nitro group, a cyano group, $-CH_2N_2$, $-CH{=}NR^1$, $-CH{=}NOR^1$ or $-CONR^1R^2$ (here, $R^1$ and $R^2$ are each a hydrogen atom or a $C_{1-4}$ alkyl group), $-COOR^3$ (here, $R^3$ is a hydrogen atom or a $C_{1-4}$ alkyl group), a substituted or unsubstituted $C_{1-6}$ normal, cyclic or branched alkyl group, a substituted or unsubstituted $C_{3-7}$ cycloalkyl group, a substituted or unsubstituted $C_{1-6}$ normal or branched alkoxyl group, a substituted or unsubstituted $C_{1-6}$ normal or branched alkylthio group, a substituted or unsubstituted $C_{1-6}$ normal or branched alkylsulfonyl group, or a substituted or unsubstituted $C_{1-6}$ normal or branched alkylsulfinyl group.

The halogen atom is an iodine atom, a fluorine atom, a chlorine atom or a bromine atom, and preferably a fluorine atom or a chlorine atom. The trihalomethyl group is specifically a trifluoromethyl group, a tribromomethyl group or a trichloromethyl group, and preferably a trifluoromethyl group. The unsubstituted $C_{1-6}$ alkyl group is specifically a methyl group, an ethyl group, a n- or i-propyl group or a cyclohexyl group, and preferably a methyl group or an ethyl group. The further preferable example is a methyl group. The unsubstituted $C_{1-6}$ alkoxyl group is specifically a methoxy group, an ethoxy group, or a n- or i-propoxy group, and preferably a methoxy group. The unsubstituted $C_{1-6}$ alkylthio group is specifically a methylthio group, an ethylthio group, or a n- or i-propylthio group, and preferably a methylthio group. The unsubstituted $C_{1-6}$ normal or branched alkylsulfonyl group is specifically a methylsulfonyl group, an ethylsulfonyl group, or a n- or i-propylsulfonyl group, and preferably a methylsulfonyl group. The unsubstituted $C_{1-6}$ normal or branched alkylsulfinyl group is specifically a methylsulfinyl group, an ethylsulfinyl group, or a n- or i-propylsulfinyl group, and preferably a methylsulfinyl group.

The substituent permissible to the groups is a halogen atom, a hydroxyl group, a nitro group, a cyano group, an acyl group, a trihalomethyl group, a trihalomethoxy group, a phenyl group, an oxo group or a phenoxy group optionally substituted with one or more halogen atoms, and the substituent may substitute singly or plurally independently at arbitrary position(s). Especially preferable substituent out of them is a halogen atom, a hydroxyl group, a cyano group or an acyl group, and further preferable substituent is a fluorine atom, a chlorine atom or a cyano group.

$R^1$ and $R^2$ are each a hydrogen atom or a $C_{1-4}$ alkyl group, and specifically a hydrogen atom, a methyl group, an ethyl group, a n- or i-propyl group, a n-, i-, s- or t-butyl group. They are each preferably a hydrogen atom, a methyl group or an ethyl group, and more preferably a hydrogen atom or a methyl group.

Especially, $X^1$ and $X^2$ are each preferably a hydrogen atom, a halogen atom, a trihalomethyl group, a cyano group, a substituted or unsubstituted $C_{1-3}$ normal or branched alkyl group, a substituted or unsubstituted $C_{1-3}$ normal or branched alkoxyl group or a substituted or unsubstituted $C_{1-3}$ normal or branched alkylthio group. More preferably, they are each a hydrogen atom, a fluorine atom, a chlorine atom, a trifluoromethyl group, a cyano group, an unsubstituted $C_{1-3}$ normal or branched alkyl group, an unsubstituted $C_{1-3}$ normal or branched alkoxyl group, or an unsubstituted $C_{1-3}$ normal or branched alkylthio group. Further preferably, they are each a hydrogen atom, a chlorine atom, a fluorine atom, a trifluoromethyl group, a cyano group, a methoxy group, an ethoxy group, a methyl group, an ethyl group or a methylthio group.

Regarding the positions of substitution of $X^1$ and $X^2$, there is no limitation; however, when the ring marked with A in the formula (1) is a benzene ring, the combination of the 5-position and the 6-position of the benzimidazole ring is the most preferable.

B is a substituted or unsubstituted $C_{1-6}$ normal, cyclic or branched alkylene group, or a substituted or unsubstituted $C_{2-6}$ normal or branched alkenylene group. The unsubstituted $C_{1-6}$ normal, cyclic or branched alkylene group is a methylene group, an ethylene group, a n- or i-propylene group, a 2,2-dimethylpropylene group, a n-, i- or t-butylene group, a 1,1-dimethylbutylene group, a n-pentylene group, a cyclohexylene group, or the like. A preferable example among them is an ethylene group, a n-propylene group or a 2,2-dimethylpropylene group, and an especially preferable example is a n-propylene group. The unsubstituted $C_{2-6}$ normal or branched alkenylene group is a vinylene group, a vinylidene group, an allylene group or the like.

Between atoms, such alkylene or alkenylene group may contain one or more of -O-, -S-, $-SO_2-$ or $-NR^{4-}$ (here, $R^4$ is a hydrogen atom or a $C_{1-6}$ normal or branched alkyl group), but said atom or atomic group does not bond directly to the M. Specifically, an example of the group formed by this is a group in which said atom or atomic group is inserted into between atoms of an ethylene group, a n-propylene group, or a n- or t-butylene group. Further specifically, it is $-CH_2OCH_2-$, $-CH_2OCH_2CH_2-$, $-CH_2SCH_2-$, $-CH_2SCH_2CH_2-$, $-CH_2SO_2CH_2-$, $-CH_2SO_2CH_2CH_2-$, $-CH_2NR^4CH_2-$, $-CH_2NR^4CH_2CH_2-$ or the like, and it is especially preferably $-CH_2OCH_2-$, $-CH_2SCH_2-$ or $-CH_2SO_2CH_2$.

The substituent permissible to the groups is a halogen atom, a hydroxyl group, a nitro group, a cyano group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched

acyl group, a $C_{1-6}$ normal or branched acylamino group, a trihalomethyl group, a trihalomethoxy group, a phenyl group, an oxo group or a phenoxy group optionally substituted with one or more halogen atoms. The substituent may each substitute singly or plurally independently at arbitrary position(s) of the alkylene or alkenylene group.

Said halogen atom is a fluorine atom, a chlorine atom, a bromine atom or an iodine atom. It is preferably a fluorine atom or a chlorine atom. Said $C_{1-6}$ normal or branched alkoxyl group is specifically a methoxy group, an ethoxy group, a n- or i-propyloxy group, a n-, i-, s- or t-butoxy group, or the like; it is preferably a methoxy group or an ethoxy group; and it is further preferably a methoxy group. Said $C_{1-6}$ normal or branched alkylthio group is specifically a methylthio group, an ethylthio group, a n- or i-propylthio group, a n-, i-, s- or t-butylthio group, or the like; it is preferably a methylthio group or an ethylthio group; and it is further preferably a methylthio group. Said $C_{1-6}$ normal or branched alkylsulfonyl group is specifically a methylsulfonyl group, an ethylsulfonyl group, a n- or i-propylsulfonyl group, a n-, i-, s- or t-butylsulfonyl group, or the like; it is preferably a methylsulfonyl group or an ethylsulfonyl group; and it is further preferably a methylsulfonyl group. Said $C_{1-6}$ normal or branched acylamino group is specifically an acetylamino group, an ethylcarbonylamino group, a n- or i-propylcarbonylamino group, or the like; it is preferably an acetylamino group or an ethylcarbonylamino group; and it is further preferably an acetylamino group. Said trihalomethyl group is specifically a trifluoromethyl group, a tribromomethyl group or a trichloromethyl group; and it is preferably a trifluoromethyl group.

Especially, B is preferably a substituted or unsubstituted $C_{1-6}$ normal,' cyclic or branched alkylene group {between atoms, the alkylene group optionally contains one or more of -O-, -S-, $-SO_2-$ or $-NR^4-$ (here, $NR_4$ is defined same as above), but the atom or the atomic group does not bond directly to the M}. More preferably, it is $-CH_2CH_2-$, $-CH_2CH_2CH_2-$, $-CH_2C(=O)CH_2-$, $-CH_2OCH_2-$, $-CH_2SCH_2-$, $-CH_2S(=O)CH_2-$, $-CH_2CF_2CH_2-$, $-CH_2SO_2CH_2-$, $-CH_2CH_2CH_2CH_2-$, $-CH_2C(CH_3)_2CH_2-$, $-CH_2SO_2CH_2CH_2-$, $-CH_2C(=O)CH_2CH_2-$, $-CH_2C(=O)C(CH_3)_2CH_2-$ or $-CH_2C(=O)C(=O)CH_2-$. Further preferably, it is $-CH_2CH_2-$, $-CH_2CH_2CH_2-$, $-CH_2C(=O)CH_2-$, $-CH_2OCH_2-$, $-CH_2SCH_2-$, $-CH_2S(=O)CH_2-$, $-CH_2CF_2CH_2-$, $-CH_2SO_2CH_2-$ or $-CH_2C(CH_3)_2CH_2-$. Further more preferably, it is $-CH_2CH_2CH_2-$ or $-CH_2C(CH_3)_2CH_2-$. Especially preferably, it is $-CH_2CH_2CH_2-$.

E expresses $-COOR^4$, $-SO_3R^4$, $-CONHR^5$, $-SO_2NHR^4$, $-PO(OR^6)_2$, a tetrazol-5-yl group, a 5-oxo-1,2,4-oxadiazol-3-yl group, or a 5-oxo-1,2,4-thiadiazol-3-yl group (here, $R^4$ is a hydrogen atom or a $C_{1-6}$ normal or branched alkyl group; $R^5$ is a hydrogen atom, a cyano group, or a $C_{1-6}$ normal or branched alkyl group; $R^6$ is a hydrogen atom, a $C_{1-6}$ normal or branched alkyl group or a trifluoromethylsulfonyl group, or a pharmaceutically permissible salt of the groups).

Here, $R^4$ is a hydrogen atom, a methyl group, an ethyl group, a n- or i-propyl group, a n-, i-, s- or t-butyl group, or the like. Preferably, it is a hydrogen atom, a methyl group or an ethyl group. Especially preferably, it is a hydrogen atom. $R^5$ is a hydrogen atom, a cyano group, a methyl group, an ethyl group, a n- or i-propyl group, a n-, i-, s- or t-butyl group, or the like. Preferably, it is a hydrogen atom, a methyl group or an ethyl group. Especially preferably, it is a hydrogen atom.

Especially, E is preferably $-COOR^4$, $-SO_3R^4$, or a tetrazol-5-yl group. Further preferably, it is $-COOR^4$. Especially preferably, it is -COOH.

G is a substituted or unsubstituted $C_{1-6}$ normal or branched alkylene group. Between atoms, the alkylene group optionally contains one or more of -O-, -S-, $-SO_2-$ or $-NR^4-$, but this atom or atomic group does not bond directly to the nitrogen atom of the imidazole ring. Further, $R^4$ is similarly defined as above. The permissible substituent to the groups is a halogen atom, a hydroxyl group, a nitro group, a cyano group, a $C_{1-6}$ normal or branched alkoxyl group (including the case where adjacent two groups form an acetal bonding), a trihalomethyl group, a trihalomethoxy group, a phenyl group or an oxo group.

Preferably, G is $-CH_2-$, $-CH_2CH_2-$, $-CH_2CO-$, $-CH_2CH_2O-$, $-CH_2CONH-$, -CO-, $-SO_2-$, $-CH_2SO_2-$, $-CH_2S-$, $-CH_2CH_2S-$ or the like (J bonds to the right side of said group). Preferably, it is $-CH_2-$, $-CH_2CH_2-$, $-CH_2CO-$ or $-CH_2CH_2O-$. Further preferably, it is $-CH_2-$.

J is a substituted or unsubstituted $C_{1-6}$ normal, cyclic or branched alkyl group, or a substituted or unsubstituted $C_{4-10}$ aryl group. The unsubstituted $C_{1-6}$ normal, cyclic or branched alkyl group is a methyl group, an ethyl group, a n- or i-propyl group, a n-, i-, s- or t-butyl group, a cyclopentyl group, a cyclohexyl group, or the like.

The substituent permissible to the groups is a halogen atom, a hydroxyl group, a nitro group, a cyano group, $-COOR^7$ (here, $R^7$ is a hydrogen atom or a $C_{1-4}$ alkyl group), a $C_{1-6}$ normal, cyclic or branched alkyl group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched alkylsulfinyl group, a $C_{1-6}$ normal or branched acyl group, a $C_{1-6}$ normal or branched acylamino group, a trihalomethyl group, a trihalomethoxy group, a phenyl group, an oxo group, or a phenoxy group optionally substituted with one or more halogen atoms. The substituents may each substitute singly or plurally independently at arbitrary position(s) of the alkyl group or aryl group. Further, the substituent is optionally substituted with a halogen atom, a hydroxyl group, a nitro group, a cyano group, an acyl group, a trihalomethyl group, a phenyl group, an oxo group or a phenoxy group optionally substituted with a halogen atom.

Especially, a preferable example of J is a substituted or unsubstituted $C_{4-10}$ aryl group. Specifically, a group

expressed by the following formula (2) or (3) is preferable.

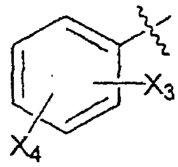

(2)

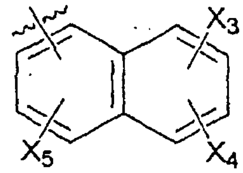

(3)

[here, $X^3$, $X^4$ and $X^5$ are each at the same time or independently a hydrogen atom, a halogen atom, a hydroxyl group, a nitro group, a cyano group, a trihalomethyl group, a trihalomethoxy group, -$COOR^7$ (here, $R^7$ is a hydrogen atom or a $C_{1-4}$ alkyl group), a substituted or unsubstituted $C_{1-3}$ normal or branched alkyl group, a substituted or unsubstituted $C_{1-3}$ normal or branched alkoxyl group, a substituted or unsubstituted $C_{1-3}$ normal or branched alkylthio group, a substituted or unsubstituted $C_{1-3}$ normal or branched alkylsulfonyl group, or a substituted or unsubstituted $C_{1-3}$ normal or branched alkylsulfinyl group; and there is no limitation regarding the substitution positions of $X^3$, $X^4$ and $X^5$ on the benzene ring or the naphthalene ring].

Said halogen atom is a fluorine atom, a chlorine atom, a bromine atom or an iodine atom. Preferably, it is a fluorine atom or a chlorine atom. The trihalomethyl group is preferably a trifluoromethyl group. The trihalomethoxy group is preferably a trifluoromethoxy group. The unsubstituted $C_{1-3}$ normal or branched alkyl group is specifically a methyl group, an ethyl group, or a n- or i-propyl group. The unsubstituted $C_{1-3}$ normal or branched alkoxyl group is specifically a methoxy group, an ethoxy group, or a n- or i-propyloxy group. The unsubstituted $C_{1-3}$ normal or branched alkylthio group is specifically a methylthio group, an ethylthio group, or a n- or i-propylthio group. The unsubstituted $C_{1-3}$ normal or branched alkylsulfonyl group is specifically a methylsulfonyl group, an ethylsulfonyl group, or a n- or i-propylsulfonyl group. The unsubstituted $C_{1-3}$ normal or branched alkylsulfinyl group is specifically a methylsulfinyl group, an ethylsulfinyl group, or a n- or i-propylsulfinyl group.

These substituents are optionally further substituted with a halogen atom, a hydroxyl group, a nitro group, a cyano group, an acyl group, a trihalomethyl group, a phenyl group, an oxo group or a phenoxy group optionally substituted with a halogen atom.

Regarding the positions of substitution of $X^3$, $X^4$ and $X^5$, there is no special limitation; however, in the above formula (2), the combination of the 2-position and the 3-position, or the combination of the 2-position and the 5-position is preferable, and in the formula (3), the combination of the 4-position, the 7-position and the 8-position, the combination of the 4-position, the 6-position and the 8-position, or the combination of the 6-position, the 7-position and the 8-position is preferable.

Especially, preferable examples of J include a 2-methylphenyl group, a 2-ethylphenyl group, a 3-trifluoromethyl group, a 2-ethoxyphenyl group, a 3-methoxyphenyl group, a 2-chlorophenyl group, a 2-trifluorophenyl group, a 2,3-methylenedioxyphenyl group, a 2-methyl-3-methoxyphenyl group, a 2-trifluoromethyl-3-methoxyphenyl group, a 2-methyl-3-trifluoromethoxyphenyl group, a 2,3-dimethylphenyl group, a 2,3-dichlorophenyl group, a 2,3-dimethoxyphenyl group, a 2,5-dimethoxyphenyl group, a 2,5-dimethylphenyl group, a 2,5-dichlorophenyl group, a 2,5-ditrifluoromethylphenyl group, a 1-naphthyl group, a 2-naphthyl group, a 8-methyl-1-naphthyl group, a 7-methyl-1-naphthyl group, a 6,8-dimethyl-1-naphthyl group and a 4,6,8-trimethyl-1-naphthyl group.

M is a sulfur atom, a sulfinyl group, a sulfonyl group, a single bond or -$CR^8R^9$- (here, $R^8$ and $R^9$ are each at the same time or independently a hydrogen atom or a $C_{1-4}$ alkyl group).

$R^8$ and $R^9$ are each a hydrogen atom, a methyl group, an ethyl group, a n- or i-propyl group, or a n-, i-, s- or t-butyl group. They are preferably hydrogen atoms.

Especially, preferable example of M is a sulfur atom, a sulfinyl group or a sulfonyl group. More preferably, M is a sulfur atom.

Further, benzimidazole derivatives expressed by the following formula (4) or their pharmaceutically permissible salts out of the compounds expressed by the above formula (1) are new compounds.

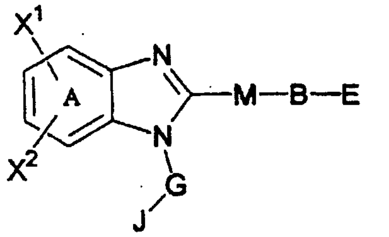

(4)

[in the formula (4), the definitions of the ring marked with A, and $X^1$, $X^2$, B, E, G, J and M are same as those in the above formula (1); however; excepting the cases where at least one of $X^1$ and $X^2$ is a cyano group, $-CH_2NH_2$, $-CH=NR^1$, $-CH=NOR^1$ or $-CONR^1R^2$ (here, $R^1$ and $R^2$ are each a hydrogen atom or a $C_{1-4}$ alkyl group), J expresses only a substituted naphthalene ring].

Regarding the substituents of the compound expressed by the above formula (4) of the present invention, the definitions and the preferable groups of $X^1$, $X^2$, G, J, M, B and E are same as those in the above formula (1).

Concrete examples of compounds expressed by the above formula (1) are shown in the following tables. Yet, B1 to B11, and J1 to J74 in the tables are groups shown in the following formulae. In the formulae, the definitions of E and G are same as their definitions shown above.

Yet, preferable compounds are those having the abovementioned preferable combinations of groups regarding $X^1$, $X^2$, the ring marked with A, and B, G, J, B and E, and not all of the compounds are shown in the tables. Further, the following tables exemplify compounds in which the ring marked with A is a benzene ring, but the situations can be considered to be same also in the case where it is a pyridine ring.

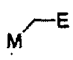
B1
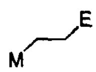
B2
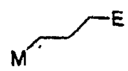
B3
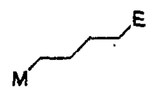
B4
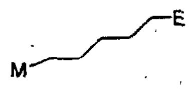
B5
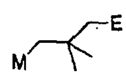
B6
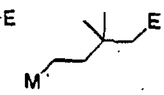
B7

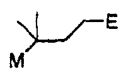
B8
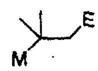
B9
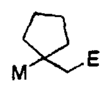
B10
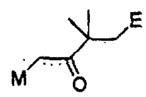
B11

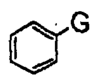
J1
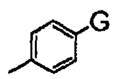
J2
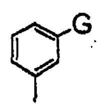
J3
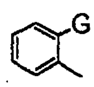
J4
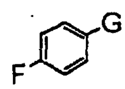
J5
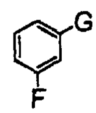
J6
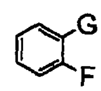
J7

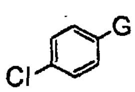
J8
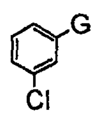
J9
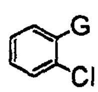
J10
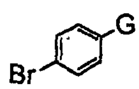
J11
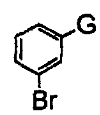
J12
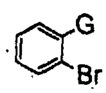
J13
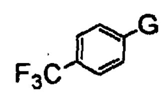
J14

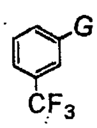
J15
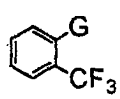
J16
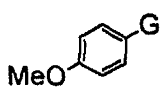
J17
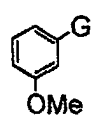
J18
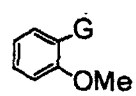
J19
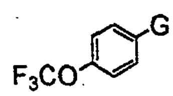
J20
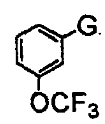
J21

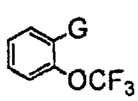
J22
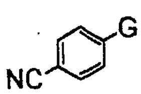
J23
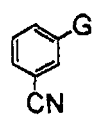
J24
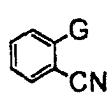
J25
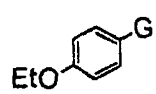
J26
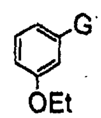
J27
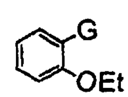
J28

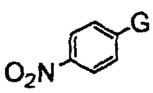
J29
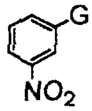
J30
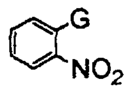
J31
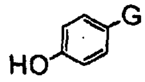
J32
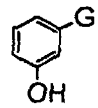
J33
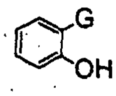
J34
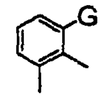
J35

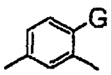
J36
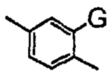
J37
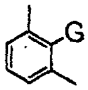
J38
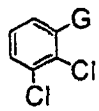
J39
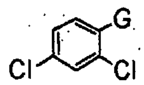
J40
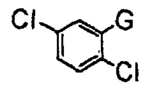
J41
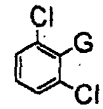
J42
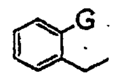
J43

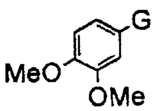
J44
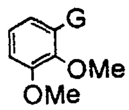
J45
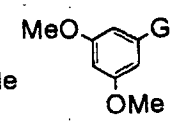
J46
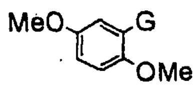
J47
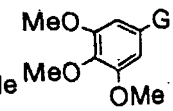
J48
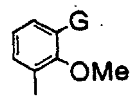
J49
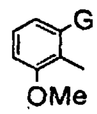
J50

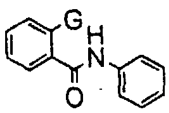
J51
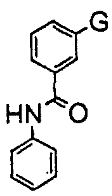
J52
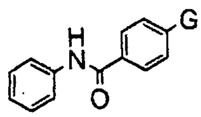
J53
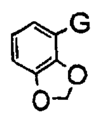
J54
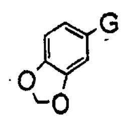
J55
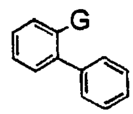
J56

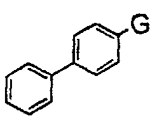
J57
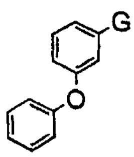
J58
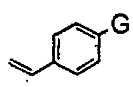
J59
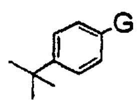
J60
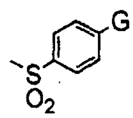
J61
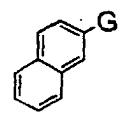
J62

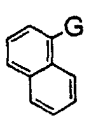
J63
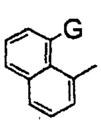
J64
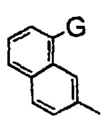
J65
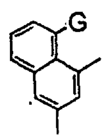
J66
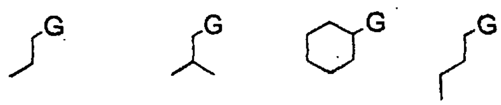
J67 J68 J69 J70

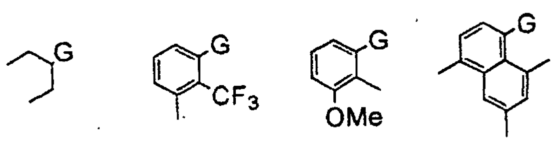
J71 J72 J73 J74

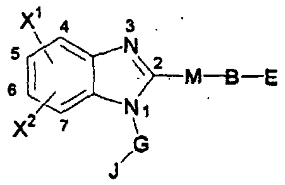

(1)

| Compound No. | $X^1$ | $X^2$ | B | E | G | J | M |
|---|---|---|---|---|---|---|---|
| 1 | H | H | B3 | COOH | $CH_2$ | J4 | S |
| 2 | 5-Me | H | B3 | COOH | $CH_2$ | J4 | S |
| 3 | 5-Et | H | B3 | COOH | $CH_2$ | J4 | S |
| 4 | 5-F | H | B3 | COOH | $CH_2$ | J4 | S |
| 5 | 5-Cl | H | B3 | COOH | $CH_2$ | J4 | S |
| 6 | 5-$CF_3$ | H | B3 | COOH | $CH_2$ | J4 | S |
| 7 | 5-MeO | H | B3 | COOH | $CH_2$ | J4 | S |
| 8 | 5-EtO | H | B3 | COOH | $CH_2$ | J4 | S |
| 9 | 5-Me | 6-Me | B3 | COOH | $CH_2$ | J4 | S |
| 10 | 5-Et | 6-Et | B3 | COOH | $CH_2$ | J4 | S |
| 11 | 5-F | 6-F | B3 | COOH | $CH_2$ | J4 | S |
| 12 | 5-Cl | 6-Cl | B3 | COOH | $CH_2$ | J4 | S |
| 13 | 5-$CF_3$ | 6-$CF_3$ | B3 | COOH | $CH_2$ | J4 | S |
| 14 | 5-MeO | 6-MeO | B3 | COOH | $CH_2$ | J4 | S |
| 15 | 5-EtO | 6-EtO | B3 | COOH | $CH_2$ | J4 | S |
| 16 | H | H | B3 | COOH | $CH_2$ | J10 | S |
| 17 | 5-Me | H | B3 | COOH | $CH_2$ | J10 | S |
| 18 | 5-Et | H | B3 | COOH | $CH_2$ | J10 | S |
| 19 | 5-F | H | B3 | COOH | $CH_2$ | J10 | S |
| 20 | 5-Cl | H | B3 | COOH | $CH_2$ | J10 | S |
| 21 | 5-$CF_3$ | H | B3 | COOH | $CH_2$ | J10 | S |
| 22 | 5-MeO | H | B3 | COOH | $CH_2$ | J10 | S |
| 23 | 5-EtO | H | B3 | COOH | $CH_2$ | J10 | S |
| 24 | 5-Me | 6-Me | B3 | COOH | $CH_2$ | J10 | S |
| 25 | 5-Et | 6-Et | B3 | COOH | $CH_2$ | J10 | S |
| 26 | 5-F | 6-F | B3 | COOH | $CH_2$ | J10 | S |
| 27 | 5-Cl | 6-Cl | B3 | COOH | $CH_2$ | J10 | S |
| 28 | 5-$CF_3$ | 6-$CF_3$ | B3 | COOH | $CH_2$ | J10 | S |
| 29 | 5-MeO | 6-MeO | B3 | COOH | $CH_2$ | J10 | S |
| 30 | 5-EtO | 6-EtO | B3 | COOH | $CH_2$ | J10 | S |
| 31 | H | H | B3 | COOH | $CH_2$ | J16 | S |
| 32 | 5-Me | H | B3 | COOH | $CH_2$ | J16 | S |

(continued)

| Compound No. | $X^1$ | $X^2$ | B | E | G | J | M |
|---|---|---|---|---|---|---|---|
| 33 | 5-Et | H | B3 | COOH | $CH_2$ | J16 | S |
| 34 | 5-F | H | B3 | COOH | $CH_2$ | J16 | S |
| 35 | 5-Cl | H | B3 | COOH | $CH_2$ | J16 | S |
| 36 | 5-$CF_3$ | H | B3 | COOH | $CH_2$ | J16 | S |
| 37 | 5-MeO | H | B3 | COOH | $CH_2$ | J16 | S |
| 38 | 5-EtO | H | B3 | COOH | $CH_2$ | J16 | S |
| 39 | 5-Me | 6-Me | B3 | COOH | $CH_2$ | J16 | S |
| 40 | 5-Et | 6-Et | B3 | COOH | $CH_2$ | J16 | S |
| 41 | 5-F | 6-F | B3 | COOH | $CH_2$ | J16 | S |
| 42 | 5-Cl | 6-Cl | B3 | COOH | $CH_2$ | J16 | S |
| 43 | 5-$CF_3$ | 6-$CF_3$ | B3 | COOH | $CH_2$ | J16 | S |
| 44 | 5-MeO | 6-MeO | B3 | COOH | $CH_2$ | J16 | S |
| 45 | 5-EtO | 6-EtO | B3 | COOH | $CH_2$ | J16 | S |
| 46 | H | H | B3 | COOH | $CH_2$ | J18 | S |
| 47 | 5-Me | H | B3 | COOH | $CH_2$ | J18 | S |
| 48 | 5-Et | H | B3 | COOH | $CH_2$ | J18 | S |
| 49 | 5-F | H | B3 | COOH | $CH_2$ | J18 | S |
| 50 | 5-Cl | H | B3 | COOH | $CH_2$ | J18 | S |
| 51 | 5-$CF_3$ | H | B3 | COOH | $CH_2$ | J18 | S |
| 52 | 5-MeO | H | B3 | COOH | $CH_2$ | J18 | S |
| 53 | 5-EtO | H | B3 | COOH | $CH_2$ | J18 | S |
| 54 | 5-Me | 6-Me | B3 | COOH | $CH_2$ | J18 | S |
| 55 | 5-Et | 6-Et | B3 | COOH | $CH_2$ | J18 | S |
| 56 | 5-F | 6-F | B3 | COOH | $CH_2$ | J18 | S |
| 57 | 5-Cl | 6-Cl | B3 | COOH | $CH_2$ | J18 | S |
| 58 | 5-$CF_3$ | 6-$CF_3$ | B3 | COOH | $CH_2$ | J18 | S |
| 59 | 5-MeO | 6-MeO | B3 | COOH | $CH_2$ | J18 | S |
| 60 | 5-EtO | 6-EtO | B3 | COOH | $CH_2$ | J18 | S |
| 61 | H | H | B3 | COOH | $CH_2$ | J21 | S |
| 62 | 5-Me | H | B3 | COOH | $CH_2$ | J21 | S |
| 63 | 5-Et | H | B3 | COOH | $CH_2$ | J21 | S |
| 64 | 5-F | H | B3 | COOH | $CH_2$ | J21 | S |
| 65 | 5-Cl | H | B3 | COOH | $CH_2$ | J21 | S |
| 66 | 5-$CF_3$ | H | B3 | COOH | $CH_2$ | J21 | S |
| 67 | 5-MeO | H | B3 | COOH | $CH_2$ | J21 | S |
| 68 | 5-EtO | H | B3 | COOH | $CH_2$ | J21 | S |
| 69 | 5-Me | 6-Me | B3 | COOH | $CH_2$ | J21 | S |
| 70 | 5-Et | 6-Et | B3 | COOH | $CH_2$ | J21 | S |

(continued)

| Compound No. | $X^1$ | $X^2$ | B | E | G | J | M |
|---|---|---|---|---|---|---|---|
| 71 | 5-F | 6-F | B3 | COOH | $CH_2$ | J21 | S |
| 72 | 5-Cl | 6-Cl | B3 | COOH | $CH_2$ | J21 | S |
| 73 | 5-$CF_3$ | 6-$CF_3$ | B3 | COOH | $CH_2$ | J21 | S |
| 74 | 5-MeO | 6-MeO | B3 | COOH | $CH_2$ | J21 | S |
| 75 | 5-EtO | 6-EtO | B3 | COOH | $CH_2$ | J21 | S |
| 76 | H | H | B3 | COOH | $CH_2$ | J38 | S |
| 77 | 5-Me | H | B3 | COOH | $CH_2$ | J38 | S |
| 78 | 5-Et | H | B3 | COOH | $CH_2$ | J38 | S |
| 79 | 5-F | H | B3 | COOH | $CH_2$ | J38 | S |
| 80 | 5-Cl | H | B3 | COOH | $CH_2$ | J38 | S |
| 81 | 5-$CF_3$ | H | B3 | COOH | $CH_2$ | J38 | S |
| 82 | 5-MeO | H | B3 | COOH | $CH_2$ | J38 | S |
| 83 | 5-EtO | H | B3 | COOH | $CH_2$ | J38 | S |
| 84 | 5-Me | 6-Me | B3 | COOH | $CH_2$ | J38 | S |
| 85 | 5-Et | 6-Et | B3 | COOH | $CH_2$ | J38 | S |
| 86 | 5-F | 6-F | B3 | COOH | $CH_2$ | J38 | S |
| 87 | 5-Cl | 6-Cl | B3 | COOH | $CH_2$ | J38 | S |
| 88 | 5-$CF_3$ | 6-$CF_3$ | B3 | COOH | $CH_2$ | J38 | S |
| 89 | 5-MeO | 6-MeO | B3 | COOH | $CH_2$ | J38 | S |
| 90 | 5-EtO | 6-EtO | B3 | COOH | $CH_2$ | J38 | S |
| 91 | H | H | B3 | COOH | $CH_2$ | J42 | S |
| 92 | 5-Me | H | B3 | COOH | $CH_2$ | J42 | S |
| 93 | 5-Et | H | B3 | COOH | $CH_2$ | J42 | S |
| 94 | 5-F | H | B3 | COOH | $CH_2$ | J42 | S |
| 95 | 5-Cl | H | B3 | COOH | $CH_2$ | J42 | S |
| 96 | 5-$CF_3$ | H | B3 | COOH | $CH_2$ | J42 | S |
| 97 | 5-MeO | H | B3 | COOH | $CH_2$ | J42 | S |
| 98 | 5-EtO | H | B3 | COOH | $CH_2$ | J42 | S |
| 99 | 5-Me | 6-Me | B3 | COOH | $CH_2$ | J42 | S |
| 100 | 5-Et | 6-Et | B3 | COOH | $CH_2$ | J42 | S |
| 101 | 5-F | 6-F | B3 | COOH | $CH_2$ | J42 | S |
| 102 | 5-Cl | 6-Cl | B3 | COOH | $CH_2$ | J42 | S |
| 103 | 5-$CF_3$ | 6-$CF_3$ | B3 | COOH | $CH_2$ | J42 | S |
| 104 | 5-MeO | 6-MeO | B3 | COOH | $CH_2$ | J42 | S |
| 105 | 5-EtO | 6-EtO | B3 | COOH | $CH_2$ | J42 | S |
| 106 | H | H | B3 | COOH | $CH_2$ | J44 | S |
| 107 | 5-Me | H | B3 | COOH | $CH_2$ | J44 | S |
| 108 | 5-Et | H | B3 | COOH | $CH_2$ | J44 | S |

(continued)

| Compound No. | $X^1$ | $X^2$ | B | E | G | J | M |
|---|---|---|---|---|---|---|---|
| 109 | 5-F | H | B3 | COOH | $CH_2$ | J44 | S |
| 110 | 5-Cl | H | B3 | COOH | $CH_2$ | J44 | S |
| 111 | 5-$CF_3$ | H | B3 | COOH | $CH_2$ | J44 | S |
| 112 | 5-MeO | H | B3 | COOH | $CH_2$ | J44 | S |
| 113 | 5-EtO | H | B3 | COOH | $CH_2$ | J44 | S |
| 114 | 5-Me | 6-Me | B3 | COOH | $CH_2$ | J44 | S |
| 115 | 5-Et | 6-Et | B3 | COOH | $CH_2$ | J44 | S |
| 116 | 5-F | 6-F | B3 | COOH | $CH_2$ | J44 | S |
| 117 | 5-Cl | 6-Cl | B3 | COOH | $CH_2$ | J44 | S |
| 118 | 5-$CF_3$ | 6-$CF_3$ | B3 | COOH | $CH_2$ | J44 | S |
| 119 | 5-MeO | 6-MeO | B3 | COOH | $CH_2$ | J44 | S |
| 120 | 5-EtO | 6-EtO | B3 | COOH | $CH_2$ | J44 | S |
| 121 | H | H | B3 | COOH | $CH_2$ | J63 | S |
| 122 | 5-Me | H | B3 | COOH | $CH_2$ | J63 | S |
| 123 | 5-Et | H | B3 | COOH | $CH_2$ | J63 | S |
| 124 | 5-F | H | B3 | COOH | $CH_2$ | J63 | S |
| 125 | 5-Cl | H | B3 | COOH | $CH_2$ | J63 | S |
| 126 | 5-$CF_3$ | H | B3 | COOH | $CH_2$ | J63 | S |
| 127 | 5-MeO | H | B3 | COOH | $CH_2$ | J63 | S |
| 128 | 5-EtO | H | B3 | COOH | $CH_2$ | J63 | S |
| 129 | 5-Me | 6-Me | B3 | COOH | $CH_2$ | J63 | S |
| 130 | 5-Et | 6-Et | B3 | COOH | $CH_2$ | J63 | S |
| 131 | 5-F | 6-F | B3 | COOH | $CH_2$ | J63 | S |
| 132 | 5-Cl | 6-Cl | B3 | COOH | $CH_2$ | J63 | S |
| 133 | 5-$CF_3$ | 6-$CF_3$ | B3 | COOH | $CH_2$ | J63 | S |
| 134 | 5-MeO | 6-MeO | B3 | COOH | $CH_2$ | J63 | S |
| 135 | 5-EtO | 6-EtO | B3 | COOH | $CH_2$ | J63 | S |
| 136 | H | H | B3 | COOH | $CH_2$ | J4 | C |
| 137 | 5-Me | H | B3 | COOH | $CH_2$ | J4 | C |
| 138 | 5-Et | H | B3 | COOH | $CH_2$ | J4 | C |
| 139 | 5-F | H | B3 | COOH | $CH_2$ | J4 | C |
| 140 | 5-Cl | H | B3 | COOH | $CH_2$ | J4 | C |
| 141 | 5-$CF_3$ | H | B3 | COOH | $CH_2$ | J4 | C |
| 142 | 5-MeO | H | B3 | COOH | $CH_2$ | J4 | C |
| 143 | 5-EtO | H | B3 | COOH | $CH_2$ | J4 | C |
| 144 | 5-Me | 6-Me | B3 | COOH | $CH_2$ | J4 | C |
| 145 | 5-Et | 6-Et | B3 | COOH | $CH_2$ | J4 | C |
| 146 | 5-F | 6-F | B3 | COOH | $CH_2$ | J4 | C |

(continued)

| Compound No. | $X^1$ | $X^2$ | B | E | G | J | M |
|---|---|---|---|---|---|---|---|
| 147 | 5-Cl | 6-Cl | B3 | COOH | $CH_2$ | J4 | C |
| 148 | 5-$CF_3$ | 6-$CF_3$ | B3 | COOH | $CH_2$ | J4 | C |
| 149 | 5-MeO | 6-MeO | B3 | COOH | $CH_2$ | J4 | C |
| 150 | 5-EtO | 6-EtO | B3 | COOH | $CH_2$ | J4 | C |
| 151 | H | H | B3 | COOH | $CH_2$ | J10 | C |
| 152 | 5-Me | H | B3 | COOH | $CH_2$ | J10 | C |
| 153 | 5-Et | H | B3 | COOH | $CH_2$ | J10 | C |
| 154 | 5-F | H | B3 | COOH | $CH_2$ | J10 | C |
| 155 | 5-Cl | H | B3 | COOH | $CH_2$ | J10 | C |
| 156 | 5-$CF_3$ | H | B3 | COOH | $CH_2$ | J10 | C |
| 157 | 5-MeO | H | B3 | COOH | $CH_2$ | J10 | C |
| 158 | 5-EtO | H | B3 | COOH | $CH_2$ | J10 | C |
| 159 | 5-Me | 6-Me | B3 | COOH | $CH_2$ | J10 | C |
| 160 | 5-Et | 6-Et | B3 | COOH | $CH_2$ | J10 | C |
| 161 | 5-F | 6-F | B3 | COOH | $CH_2$ | J10 | C |
| 162 | 5-Cl | 6-Cl | B3 | COOH | $CH_2$ | J10 | C |
| 163 | 5-$CF_3$ | 6-$CF_3$ | B3 | COOH | $CH_2$ | J10 | C |
| 164 | 5-MeO | 6-MeO | B3 | COOH | $CH_2$ | J10 | C |
| 165 | 5-EtO | 6-EtO | B3 | COOH | $CH_2$ | J10 | C |
| 166 | H | H | B3 | COOH | $CH_2$ | J16 | C |
| 167 | 5-Me | H | B3 | COOH | $CH_2$ | J16 | C |
| 168 | 5-Et | H | B3 | COOH | $CH_2$ | J16 | C |
| 169 | 5-F | H | B3 | COOH | $CH_2$ | J16 | C |
| 170 | 5-Cl | H | B3 | COOH | $CH_2$ | J16 | C |
| 171 | 5-$CF_3$ | H | B3 | COOH | $CH_2$ | J16 | C |
| 172 | 5-MeO | H | B3 | COOH | $CH_2$ | J16 | C |
| 173 | 5-EtO | H | B3 | COOH | $CH_2$ | J16 | C |
| 174 | 5-Me | 6-Me | B3 | COOH | $CH_2$ | J16 | C |
| 175 | 5-Et | 6-Et | B3 | COOH | $CH_2$ | J16 | C |
| 176 | 5-F | 6-F | B3 | COOH | $CH_2$ | J16 | C |
| 177 | 5-Cl | 6-Cl | B3 | COOH | $CH_2$ | J16 | C |
| 178 | 5-$CF_3$ | 6-$CF_3$ | B3 | COOH | $CH_2$ | J16 | C |
| 179 | 5-MeO | 6-MeO | B3 | COOH | $CH_2$ | J16 | C |
| 180 | 5-EtO | 6-EtO | B3 | COOH | $CH_2$ | J16 | C |
| 181 | H | H | B3 | COOH | $CH_2$ | J18 | C |
| 182 | 5-Me | H | B3 | COOH | $CH_2$ | J18 | C |
| 183 | 5-Et | H | B3 | COOH | $CH_2$ | J18 | C |
| 184 | 5-F | H | B3 | COOH | $CH_2$ | J18 | C |

(continued)

| Compound No. | $X^1$ | $X^2$ | B | E | G | J | M |
|---|---|---|---|---|---|---|---|
| 185 | 5-Cl | H | B3 | COOH | $CH_2$ | J18 | C |
| 186 | 5-$CF_3$ | H | B3 | COOH | $CH_2$ | J18 | C |
| 187 | 5-MeO | H | B3 | COOH | $CH_2$ | J18 | C |
| 188 | 5-EtO | H | B3 | COOH | $CH_2$ | J18 | C |
| 189 | 5-Me | 6-Me | B3 | COOH | $CH_2$ | J18 | C |
| 190 | 5-Et | 6-Et | B3 | COOH | $CH_2$ | J18 | C |
| 191 | 5-F | 6-F | B3 | COOH | $CH_2$ | J18 | C |
| 192 | 5-Cl | 6-Cl | B3 | COOH | $CH_2$ | J18 | C |
| 193 | 5-$CF_3$ | 6-$CF_3$ | B3 | COOH | $CH_2$ | J18 | C |
| 194 | 5-MeO | 6-MeO | B3 | COOH | $CH_2$ | J18 | C |
| 195 | 5-EtO | 6-EtO | B3 | COOH | $CH_2$ | J18 | C |
| 196 | H | H | B3 | COOH | $CH_2$ | J21 | C |
| 197 | 5-Me | H | B3 | COOH | $CH_2$ | J21 | C |
| 198 | 5-Et | H | B3 | COOH | $CH_2$ | J21 | C |
| 199 | 5-F | H | B3 | COOH | $CH_2$ | J21 | C |
| 200 | 5-Cl | H | B3 | COOH | $CH_2$ | J21 | C |
| 201 | 5-$CF_3$ | H | B3 | COOH | $CH_2$ | J21 | C |
| 202 | 5-MeO | H | B3 | COOH | $CH_2$ | J21 | C |
| 203 | 5-EtO | H | B3 | COOH | $CH_2$ | J21 | C |
| 204 | 5-Me | 6-Me | B3 | COOH | $CH_2$ | J21 | C |
| 205 | 5-Et | 6-Et | B3 | COOH | $CH_2$ | J21 | C |
| 206 | 5-F | 6-F | B3 | COOH | $CH_2$ | J21 | C |
| 207 | 5-Cl | 6-Cl | B3 | COOH | $CH_2$ | J21 | C |
| 208 | 5-$CF_3$ | 6-$CF_3$ | B3 | COOH | $CH_2$ | J21 | C |
| 209 | 5-MeO | 6-MeO | B3 | COOH | CH2 | J21 | C |
| 210 | 5-EtO | 6-EtO | B3 | COOH | $CH_2$ | J21 | C |
| 211 | H | H | B3 | COOH | $CH_2$ | J38 | C |
| 212 | 5-Me | H | B3 | COOH | $CH_2$ | J38 | C |
| 213 | 5-Et | H | B3 | COOH | $CH_2$ | J38 | C |
| 214 | 5-F | H | B3 | COOH | $CH_2$ | J38 | C |
| 215 | 5-Cl | H | B3 | COOH | $CH_2$ | J38 | C |
| 216 | 5-$CF_3$ | H | B3 | COOH | $CH_2$ | J38 | C |
| 217 | 5-MeO | H | B3 | COOH | $CH_2$ | J38 | C |
| 218 | 5-EtO | H | B3 | COOH | $CH_2$ | J38 | C |
| 219 | 5-Me | 6-Me | B3 | COOH | $CH_2$ | J38 | C |
| 220 | 5-Et | 6-Et | B3 | COOH | $CH_2$ | J38 | C |
| 221 | 5-F | 6-F | B3 | COOH | $CH_2$ | J38 | C |
| 222 | 5-Cl | 6-Cl | B3 | COOH | $CH_2$ | J38 | C |

(continued)

| Compound No. | $X^1$ | $X^2$ | B | E | G | J | M |
|---|---|---|---|---|---|---|---|
| 223 | 5-$CF_3$ | 6-$CF_3$ | B3 | COOH | $CH_2$ | J38 | C |
| 224 | 5-MeO | 6-MeO | B3 | COOH | $CH_2$ | J38 | C |
| 225 | 5-EtO | 6-EtO | B3 | COOH | $CH_2$ | J38 | C |
| 226 | H | H | B3 | COOH | $CH_2$ | J42 | C |
| 227 | 5-Me | H | B3 | COOH | $CH_2$ | J42 | C |
| 228 | 5-Et | H | B3 | COOH | $CH_2$ | J42 | C |
| 229 | 5-F | H | B3 | COOH | $CH_2$ | J42 | C |
| 230 | 5-Cl | H | B3 | COOH | $CH_2$ | J42 | C |
| 231 | 5-$CF_3$ | H | B3 | COOH | $CH_2$ | J42 | C |
| 232 | 5-MeO | H | B3 | COOH | $CH_2$ | J42 | C |
| 233 | 5-EtO | H | B3 | COOH | $CH_2$ | J42 | C |
| 234 | 5-Me | 6-Me | B3 | COOH | $CH_2$ | J42 | C |
| 235 | 5-Et | 6-Et | B3 | COOH | $CH_2$ | J42 | C |
| 236 | 5-F | 6-F | B3 | COOH | $CH_2$ | J42 | C |
| 237 | 5-Cl | 6-Cl | B3 | COOH | $CH_2$ | J42 | C |
| 238 | 5-$CF_3$ | 6-$CF_3$ | B3 | COOH | $CH_2$ | J42 | C |
| 239 | 5-MeO | 6-MeO | B3 | COOH | $CH_2$ | J42 | C |
| 240 | 5-EtO | 6-EtO | B3 | COOH | $CH_2$ | J42 | C |
| 241 | H | H | B3 | COOH | $CH_2$ | J44 | C |
| 242 | 5-Me | H | B3 | COOH | $CH_2$ | J44 | C |
| 243 | 5-Et | H | B3 | COOH | $CH_2$ | J44 | C |
| 244 | 5-F | H | B3 | COOH | $CH_2$ | J44 | C |
| 245 | 5-Cl | H | B3 | COOH | $CH_2$ | J44 | C |
| 246 | 5-$CF_3$ | H | B3 | COOH | $CH_2$ | J44 | C |
| 247 | 5-MeO | H | B3 | COOH | $CH_2$ | J44 | C |
| 248 | 5-EtO | H | B3 | COOH | $CH_2$ | J44 | C |
| 249 | 5-Me | 6-Me | B3 | COOH | $CH_2$ | J44 | C |
| 250 | 5-Et | 6-Et | B3 | COOH | $CH_2$ | J44 | C |
| 251 | 5-F | 6-F | B3 | COOH | $CH_2$ | J44 | C |
| 252 | 5-Cl | 6-Cl | B3 | COOH | $CH_2$ | J44 | C |
| 253 | 5-$CF_3$ | 6-$CF_3$ | B3 | COOH | $CH_2$ | J44 | C |
| 254 | 5-MeO | 6-MeO | B3 | COOH | $CH_2$ | J44 | C |
| 255 | 5-EtO | 6-EtO | B3 | COOH | $CH_2$ | J44 | C |
| 256 | H | H | B3 | COOH | $CH_2$ | J63 | C |
| 257 | 5-Me | H | B3 | COOH | $CH_2$ | J63 | C |
| 258 | 5-Et | H | B3 | COOH | $CH_2$ | J63 | C |
| 259 | 5-F | H | B3 | COOH | $CH_2$ | J63 | C |
| 260 | 5-Cl | H | B3 | COOH | G | J63 | C |

(continued)

| Compound No. | $X^1$ | $X^2$ | B | E | G | J | M |
|---|---|---|---|---|---|---|---|
| 261 | 5-$CF_3$ | H | B3 | COOH | $CH_2$ | J63 | C |
| 262 | 5-MeO | H | B3 | COOH | $CH_2$ | J63 | C |
| 263 | 5-EtO | H | B3 | COOH | $CH_2$ | J63 | C |
| 264 | 5-Me | 6-Me | B3 | COOH | $CH_2$ | J63 | C |
| 265 | 5-Et | 6-Et | B3 | COOH | $CH_2$ | J63 | C |
| 266 | 5-F | 6-F | B3 | COOH | $CH_2$ | J63 | C |
| 267 | 5-Cl | 6-Cl | B3 | COOH | $CH_2$ | J63 | C |
| 268 | 5-$CF_3$ | 6-$CF_3$ | B3 | COOH | $CH_2$ | J63 | C |
| 269 | 5-MeO | 6-MeO | B3 | COOH | $CH_2$ | J63 | C |
| 270 | 5-EtO | 6-EtO | B3 | COOH | $CH_2$ | J63 | C |
| 271 | H | H | B9 | COOH | $CH_2$ | J64 | C |
| 272 | 5-Me | H | B9 | COOH | $CH_2$ | J64 | C |
| 273 | 5-Et | H | B9 | COOH | $CH_2$ | J64 | C |
| 274 | 5-F | H | B9 | COOH | $CH_2$ | J64 | C |
| 275 | 5-Cl | H | B9 | COOH | $CH_2$ | J64 | C |
| 276 | 5-$CF_3$ | H | B9 | COOH | $CH_2$ | J64 | C |
| 277 | 5-MeO | H | B9 | COOH | $CH_2$ | J64 | C |
| 278 | 5-EtO | H | B9 | COOH | $CH_2$ | J64 | C |
| 279 | 5-Me | 6-Me | B9 | COOH | $CH_2$ | J64 | C |
| 280 | 5-Et | 6-Et | B9 | COOH | $CH_2$ | J64 | C |
| 281 | 5-F | 6-F | B9 | COOH | $CH_2$ | J64 | C |
| 282 | 5-Cl | 6-Cl | B9 | COOH | $CH_2$ | J64 | C |
| 283 | 5-$CF_3$ | 6-$CF_3$ | B9 | COOH | $CH_2$ | J64 | C |
| 284 | 5-MeO | 6-MeO | B9 | COOH | $CH_2$ | J64 | C |
| 285 | 5-EtO | 6-EtO | B9 | COOH | $CH_2$ | J64 | C |
| 286 | H | H | B10 | COOH | $CH_2$ | J63 | C |
| 287 | 5-Me | H | B10 | COOH | $CH_2$ | J63 | C |
| 288 | 5-Et | H | B10 | COOH | $CH_2$ | J63 | C |
| 289 | 5-F | H | B10 | COOH | $CH_2$ | J63 | C |
| 290 | 5-Cl | H | B10 | COOH | $CH_2$ | J63 | C |
| 291 | 5-$CF_3$ | H | B10 | COOH | $CH_2$ | J63 | C |
| 292 | 5-MeO | H | B10 | COOH | $CH_2$ | J63 | C |
| 293 | 5-EtO | H | B10 | COOH | $CH_2$ | J63 | C |
| 294 | 5-Me | 6-Me | B10 | COOH | $CH_2$ | J63 | C |
| 295 | 5-Et | 6-Et | B10 | COOH | $CH_2$ | J63 | C |
| 296 | 5-F | 6-F | B10 | COOH | $CH_2$ | J63 | C |
| 297 | 5-Cl | 6-Cl | B10 | COOH | $CH_2$ | J63 | C |
| 298 | 5-$CF_3$ | 6-$CF_3$ | B10 | COOH | $CH_2$ | J63 | C |

(continued)

| Compound No. | $X^1$ | $X^2$ | B | E | G | J | M |
|---|---|---|---|---|---|---|---|
| 299 | 5-MeO | 6-MeO | B10 | COOH | $CH_2$ | J63 | C |
| 300 | 5-EtO | 6-EtO | B10 | COOH | $CH_2$ | J63 | C |
| 301 | 5-CN | H | B3 | COOH | $CH_2$ | J1 | S |
| 302 | 5-CN | H | B3 | COOH | $CH_2$ | J2 | S |
| 303 | 5-CN | H | B3 | COOH | $CH_2$ | J3 | S |
| 304 | 5-CN | H | B3 | COOH | $CH_2$ | J4 | S |
| 305 | 5-CN | H | B3 | COOH | $CH_2$ | J5 | S |
| 306 | 5-CN | H | B3 | COOH | $CH_2$ | J6 | S |
| 307 | 5-CN | H | B3 | COOH | $CH_2$ | J7 | S |
| 308 | 5-CN | H | B3 | COOH | $CH_2$ | J8 | S |
| 309 | 5-CN | H | B3 | COOH | $CH_2$ | J9 | S |
| 310 | 5-CN | H | B3 | COOH | $CH_2$ | J10 | S |
| 311 | 5-CN | H | B3 | COOH | $CH_2$ | J11 | S |
| 312 | 5-CN | H | B3 | COOH | $CH_2$ | J12 | S |
| 313 | 5-CN | H | B3 | COOH | $CH_2$ | J13 | S |
| 314 | 5-CN | H | B3 | COOH | $CH_2$ | J14 | S |
| 315 | 5-CN | H | B3 | COOH | $CH_2$ | J15 | S |
| 316 | 5-CN | H | B3 | COOH | $CH_2$ | J16 | S |
| 317 | 5-CN | H | B3 | COOH | $CH_2$ | J17 | S |
| 318 | 5-CN | H | B3 | COOH | $CH_2$ | J18 | S |
| 319 | 5-CN | H | B3 | COOH | $CH_2$ | J19 | S |
| 320 | 5-CN | H | B3 | COOH | $CH_2$ | J20 | S |
| 321 | 5-CN | H | B3 | COOH | $CH_2$ | J21 | S |
| 322 | 5-CN | H | B3 | COOH | $CH_2$ | J22 | S |
| 323 | 5-CN | H | B3 | COOH | $CH_2$ | J23 | S |
| 324 | 5-CN | H | B3 | COOH | $CH_2$ | J24 | S |
| 325 | 5-CN | H | B3 | COOH | $CH_2$ | J25 | S |
| 326 | 5-CN | H | B3 | COOH | $CH_2$ | J26 | S |
| 327 | 5-CN | H | B3 | COOH | $CH_2$ | J27 | S |
| 328 | 5-CN | H | B3 | COOH | $CH_2$ | J28 | S |
| 329 | 5-CN | H | B3 | COOH | $CH_2$ | J29 | S |
| 330 | 5-CN | H | B3 | COOH | $CH_2$ | J30 | S |
| 331 | 5-CN | H | B3 | COOH | $CH_2$ | J31 | S |
| 332 | 5-CN | H | B3 | COOH | $CH_2$ | J32 | S |
| 333 | 5-CN | H | B3 | COOH | $CH_2$ | J33 | S |
| 334 | 5-CN | H | B3 | COOH | $CH_2$ | J34 | S |
| 335 | 5-CN | H | B3 | COOH | $CH_2$ | J35 | S |
| 336 | 5-CN | H | B3 | COOH | $CH_2$ | J36 | S |

(continued)

| Compound No. | $X^1$ | $X^2$ | B | E | G | J | M |
|---|---|---|---|---|---|---|---|
| 337 | 5-CN | H | B3 | COOH | $CH_2$ | J37 | S |
| 338 | 5-CN | H | B3 | COOH | $CH_2$ | J38 | S |
| 339 | 5-CN | H | B3 | COOH | $CH_2$ | J39 | S |
| 340 | 5-CN | H | B3 | COOH | $CH_2$ | J40 | S |
| 341 | 5-CN | H | B3 | COOH | $CH_2$ | J41 | S |
| 342 | 5-CN | H | B3 | COOH | $CH_2$ | J42 | S |
| 343 | 5-CN | H | B3 | COOH | $CH_2$ | J43 | S |
| 344 | 5-CN | H | B3 | COOH | $CH_2$ | J44 | S |
| 345 | 5-CN | H | B3 | COOH | $CH_2$ | J45 | S |
| 346 | 5-CN | H | B3 | COOH | $CH_2$ | J46 | S |
| 347 | 5-CN | H | B3 | COOH | $CH_2$ | J47 | S |
| 348 | 5-CN | H | B3 | COOH | $CH_2$ | J48 | S |
| 349 | 5-CN | H | B3 | COOH | $CH_2$ | J49 | S |
| 350 | 5-CN | H | B3 | COOH | $CH_2$ | J50 | S |
| 351 | 5-CN | H | B3 | COOH | $CH_2$ | J51 | S |
| 352 | 5-CN | H | B3 | COOH | $CH_2$ | J52 | S |
| 353 | 5-CN | H | B3 | COOH | $CH_2$ | J53 | S |
| 354 | 5-CN | H | B3 | COOH | $CH_2$ | J54 | S |
| 355 | 5-CN | H | B3 | COOH | $CH_2$ | J55 | S |
| 356 | 5-CN | H | B3 | COOH | $CH_2$ | J56 | S |
| 357 | 5-CN | H | B3 | COOH | $CH_2$ | J57 | S |
| 358 | 5-CN | H | B3 | COOH | $CH_2$ | J58 | S |
| 359 | 5-CN | H | B3 | COOH | $CH_2$ | J59 | S |
| 360 | 5-CN | H | B3 | COOH | $CH_2$ | J60 | S |
| 361 | 5-CN | H | B3 | COOH | $CH_2$ | J61 | S |
| 362 | 5-CN | H | B3 | COOH | $CH_2$ | J62 | S |
| 363 | 5-CN | H | B3 | COOH | $CH_2$ | J63 | S |
| 364 | 5-CN | H | B3 | COOH | $CH_2$ | J64 | S |
| 365 | 5-CN | H | B3 | COOH | $CH_2$ | J65 | S |
| 366 | 5-CN | H | B3 | COOH | $CH_2$ | J66 | S |
| 367 | 5-CN | H | B3 | COOH | $CH_2$ | J67 | S |
| 368 | 5-CN | H | B3 | COOH | $CH_2$ | J68 | S |
| 369 | 5-CN | H | B3 | COOH | $CH_2$ | J69 | S |
| 370 | 5-CN | H | B1 | COOH | $CH_2$ | J4 | S |
| 371 | 5-CN | H | B2 | COOH | $CH_2$ | J4 | S |
| 372 | 5-CN | H | B4 | COOH | $CH_2$ | J4 | S |
| 373 | 5-CN | H | B5 | COOH | $CH_2$ | J4 | S |
| 374 | 5-CN | H | B1 | COOH | $CH_2$ | J10 | S |

(continued)

| Compound No. | $X^1$ | $X^2$ | B | E | G | J | M |
|---|---|---|---|---|---|---|---|
| 375 | 5-CN | H | B2 | COOH | $CH_2$ | J10 | S |
| 376 | 5-CN | H | B4 | COOH | $CH_2$ | J10 | S |
| 377 | 5-CN | H | B5 | COOH | $CH_2$ | J10 | S |
| 378 | 5-CN | H | B1 | COOH | $CH_2$ | J13 | S |
| 379 | 5-CN | H | B2 | COOH | $CH_2$ | J13 | S |
| 380 | 5-CN | H | B4 | COOH | $CH_2$ | J13 | S |
| 381 | 5-CN | H | B5 | COOH | $CH_2$ | J13 | S |
| 382 | 5-CN | H | B1 | COOH | $CH_2$ | J16 | S |
| 383 | 5-CN | H | B2 | COOH | $CH_2$ | J16 | S |
| 384 | 5-CN | H | B4 | COOH | $CH_2$ | J16 | S |
| 385 | 5-CN | H | B5 | COOH | $CH_2$ | J16 | S |
| 386 | 5-CN | H | B1 | COOH | $CH_2$ | J18 | S |
| 387 | 5-CN | H | B2 | COOH | $CH_2$ | J18 | S |
| 388 | 5-CN | H | B4 | COOH | $CH_2$ | J18 | S |
| 389 | 5-CN | H | B5 | COOH | $CH_2$ | J18 | S |
| 390 | 5-CN | H | B1 | COOH | $CH_2$ | J21 | S |
| 391 | 5-CN | H | B2 | COOH | $CH_2$ | J21 | S |
| 392 | 5-CN | H | B4 | COOH | $CH_2$ | J21 | S |
| 393 | 5-CN | H | B5 | COOH | $CH_2$ | J21 | S |
| 394 | 5-CN | H | B1 | COOH | $CH_2$ | J35 | S |
| 395 | 5-CN | H | B2 | COOH | $CH_2$ | J35 | S |
| 396 | 5-CN | H | B4 | COOH | $CH_2$ | J35 | S |
| 397 | 5-CN | H | B5 | COOH | $CH_2$ | J35 | S |
| 398 | 5-CN | H | B1 | COOH | $CH_2$ | J37 | S |
| 399 | 5-CN | H | B2 | COOH | $CH_2$ | J37 | S |
| 400 | 5-CN | H | B4 | COOH | $CH_2$ | J37 | S |
| 401 | 5-CN | H | B5 | COOH | $CH_2$ | J37 | S |
| 402 | 5-CN | H | B1 | COOH | $CH_2$ | J39 | S |
| 403 | 5-CN | H | B2 | COOH | $CH_2$ | J39 | S |
| 404 | 5-CN | H | B4 | COOH | $CH_2$ | J39 | S |
| 405 | 5-CN | H | B5 | COOH | $CH_2$ | J39 | S |
| 406 | 5-CN | H | B1 | COOH | $CH_2$ | J41 | S |
| 407 | 5-CN | H | B2 | COOH | $CH_2$ | J41 | S |
| 408 | 5-CN | H | B4 | COOH | $CH_2$ | J41 | S |
| 409 | 5-CN | H | B5 | COOH | $CH_2$ | J41 | S |
| 410 | 5-CN | H | B1 | COOH | $CH_2$ | J45 | S |
| 411 | 5-CN | H | B2 | COOH | $CH_2$ | J45 | S |
| 412 | 5-CN | H | B4 | COOH | $CH_2$ | J45 | S |

(continued)

| Compound No. | $X^1$ | $X^2$ | B | E | G | J | M |
|---|---|---|---|---|---|---|---|
| 413 | 5-CN | H | B5 | COOH | $CH_2$ | J45 | S |
| 414 | 5-CN | H | B1 | COOH | $CH_2$ | J47 | S |
| 415 | 5-CN | H | B2 | COOH | $CH_2$ | J47 | S |
| 416 | 5-CN | H | B4 | COOH | $CH_2$ | J47 | S |
| 417 | 5-CN | H | B5 | COOH | $CH_2$ | J47 | S |
| 418 | 5-CN | H | B1 | COOH | $CH_2$ | J50 | S |
| 419 | 5-CN | H | B2 | COOH | $CH_2$ | J50 | S |
| 420 | 5-CN | H | B4 | COOH | $CH_2$ | J50 | S |
| 421 | 5-CN | H | B5 | COOH | $CH_2$ | J50 | S |
| 422 | 5-CN | H | B1 | COOH | $CH_2$ | J63 | S |
| 423 | 5-CN | H | B2 | COOH | $CH_2$ | J63 | S |
| 424 | 5-CN | H | B4 | COOH | $CH_2$ | J63 | S |
| 425 | 5-CN | H | B5 | COOH | $CH_2$ | J63 | S |
| 426 | 5-CN | H | B1 | COOH | $CH_2$ | J63 | SO |
| 427 | 5-CN | H | B2 | COOH | $CH_2$ | J63 | SO |
| 428 | 5-CN | H | B3 | COOH | $CH_2$ | J63 | SO |
| 429 | 5-CN | H | B4 | COOH | $CH_2$ | J63 | SO |
| 430 | 5-CN | H | B5 | COOH | $CH_2$ | J63 | SO |
| 431 | 5-CN | H | B1 | COOH | $CH_2$ | J63 | $SO_2$ |
| 432 | 5-CN | H | B2 | COOH | $CH_2$ | J63 | $SO_2$ |
| 433 | 5-CN | H | B3 | COOH | $CH_2$ | J63 | $SO_2$ |
| 434 | 5-CN | H | B4 | COOH | $CH_2$ | J63 | $SO_2$ |
| 435 | 5-CN | H | B5 | COOH | $CH_2$ | J63 | $SO_2$ |
| 436 | 5-CN | H | B1 | COOH | $CH_2$ | J63 | single bond |
| 437 | 5-CN | H | B2 | COOH | $CH_2$ | J63 | single bond |
| 438 | 5-CN | H | B3 | COOH | $CH_2$ | J63 | single bond |
| 439 | 5-CN | H | B4 | COOH | $CH_2$ | J63 | single bond |
| 440 | 5-CN | H | B5 | COOH | $CH_2$ | J63 | single bond |
| 441 | 5-CN | H | B6 | COOH | $CH_2$ | J4 | S |
| 442 | 5-CN | H | B6 | COOH | $CH_2$ | J10 | S |
| 443 | 5-CN | H | B6 | COOH | $CH_2$ | J13 | S |
| 444 | 5-CN | H | B6 | COOH | $CH_2$ | J16 | S |
| 445 | 5-CN | H | B6 | COOH | $CH_2$ | J18 | S |
| 446 | 5-CN | H | B6 | COOH | $CH_2$ | J21 | S |
| 447 | 5-CN | H | B6 | COOH | $CH_2$ | J35 | S |
| 448 | 5-CN | H | B6 | COOH | $CH_2$ | J37 | S |
| 449 | 5-CN | H | B6 | COOH | $CH_2$ | J39 | S |
| 450 | 5-CN | H | B6 | COOH | $CH_2$ | J41 | S |

(continued)

| Compound No. | $X^1$ | $X^2$ | B | E | G | J | M |
|---|---|---|---|---|---|---|---|
| 451 | 5-CN | H | B6 | COOH | $CH_2$ | J45 | S |
| 452 | 5-CN | H | B6 | COOH | $CH_2$ | J47 | S |
| 453 | 5-CN | H | B6 | COOH | $CH_2$ | J50 | S |
| 454 | 5-CN | H | B6 | COOH | $CH_2$ | J63 | S |
| 455 | 5-CN | H | B6 | COOH | $CH_2$ | J63 | SO |
| 456 | 5-CN | H | B6 | COOH | $CH_2$ | J63 | $SO_2$ |
| 457 | 5-CN | 457 | B6 | COOH | $CH_2$ | J63 | single bond |
| 458 | 5-CN | H | B7 | COOH | $CH_2$ | J4 | S |
| 459 | 5-CN | H | B7 | COOH | $CH_2$ | J10 | S |
| 460 | 5-CN | H | B7 | COOH | $CH_2$ | J13 | S |
| 461 | 5-CN | H | B7 | COOH | $CH_2$ | J16 | S |
| 462 | 5-CN | H | B7 | COOH | $CH_2$ | J18 | S |
| 463 | 5-CN | H | B7 | COOH | $CH_2$ | J21 | S |
| 464 | 5-CN | H | B7 | COOH | $CH_2$ | J35 | S |
| 465 | 5-CN | H | B7 | COOH | $CH_2$ | J37 | S |
| 466 | 5-CN | H | B7 | COOH | $CH_2$ | J39 | S |
| 467 | 5-CN | H | B7 | COOH | $CH_2$ | J41 | S |
| 468 | 5-CN | H | B7 | COOH | $CH_2$ | J45 | S |
| 469 | 5-CN | H | B7 | COOH | $CH_2$ | J47 | S |
| 470 | 5-CN | H | B7 | COOH | $CH_2$ | J50 | S |
| 471 | 5-CN | H | B7 | COOH | $CH_2$ | J63 | S |
| 472 | 5-CN | H | B7 | COOH | $CH_2$ | J63 | SO |
| 473 | 5-CN | H | B7 | COOH | $CH_2$ | J63 | $SO_2$ |
| 474 | 5-CN | H | B7 | COOH | $CH_2$ | J63 | single bond |
| 475 | 5-CN | H | B8 | COOH | $CH_2$ | J10 | S |
| 476 | 5-CN | H | B8 | COOH | $CH_2$ | J13 | S |
| 477 | 5-CN | H | B8 | COOH | $CH_2$ | J13 | S |
| 478 | 5-CN | H | B8 | COOH | $CH_2$ | J16 | S |
| 479 | 5-CN | H | B8 | COOH | $CH_2$ | J18 | S |
| 480 | 5-CN | H | B8 | COOH | $CH_2$ | J21 | S |
| 481 | 5-CN | H | B8 | COOH | $CH_2$ | J35 | S |
| 482 | 5-CN | H | B8 | COOH | $CH_2$ | J37 | S |
| 483 | 5-CN | H | B8 | COOH | $CH_2$ | J39 | S |
| 484 | 5-CN | H | B8 | COOH | $CH_2$ | J41 | S |
| 485 | 5-CN | H | B8 | COOH | $CH_2$ | J45 | S |
| 486 | 5-CN | H | B8 | COOH | $CH_2$ | J47 | S |
| 487 | 5-CN | H | B8 | COOH | $CH_2$ | J50 | S |
| 488 | 5-CN | H | B8 | COOH | $CH_2$ | J63 | S |

(continued)

| Compound No. | $X^1$ | $X^2$ | B | E | G | J | M |
|---|---|---|---|---|---|---|---|
| 489 | 5-CN | H | B8 | COOH | $CH_2$ | J63 | SO |
| 490 | 5-CN | H | B8 | COOH | $CH_2$ | J63 | $SO_2$ |
| 491 | 5-CN | H | B8 | COOH | $CH_2$ | J63 | single bond |
| 492 | H | H | B3 | COOH | $CH_2$ | J64 | S |
| 493 | 5-Me | 6-Me | B3 | COOH | $CH_2$ | J64 | S |
| 494 | H | H | B11 | COOH | $CH_2$ | J63 | S |
| 495 | 5-MeO | H | B3 | COOH | $CH_2$ | J37 | S |
| 496 | H | H | B2 | COOH | $CH_2$ | J63 | S |
| 497 | H | H | B4 | COOH | $CH_2$ | J63 | S |
| 498 | 5-Me | 6-Me | B3 | COOH | $CH_2$ | J63 | $SO_2$ |
| 499 | H | H | B3 | COOH | $CH_2$ | J3 | S |
| 500 | 5-Me | H | B3 | COOH | $CH_2$ | J3 | S |
| 501 | 5-Et | H | B3 | COOH | $CH_2$ | J3 | S |
| 502 | 5-F | H | B3 | COOH | $CH_2$ | J3 | S |
| 503 | 5-Cl | H | B3 | COOH | $CH_2$ | J3 | S |
| 504 | 5-$CF_3$ | H | B3 | COOH | $CH_2$ | J3 | S |
| 505 | 5-MeO | H | B3 | COOH | $CH_2$ | J3 | S |
| 506 | 5-EtO | H | B3 | COOH | $CH_2$ | J3 | S |
| 507 | 5-Me | 6-Me | B3 | COOH | $CH_2$ | J3 | S |
| 508 | 5-Et | 6-Et | B3 | COOH | $CH_2$ | J3 | S |
| 509 | 5-F | 6-F | B3 | COOH | $CH_2$ | J3 | S |
| 510 | 5-Cl | 6-Cl | B3 | COOH | $CH_2$ | J3 | S |
| 511 | 5-$CF_3$ | 6-$CF_3$ | B3 | COOH | $CH_2$ | J3 | S |
| 512 | 5-MeO | 6-MeO | B3 | COOH | $CH_2$ | J3 | S |
| 513 | 5-EtO | 6-EtO | B3 | COOH | $CH_2$ | J3 | S |
| 514 | H | H | B3 | COOH | $CH_2$ | J15 | S |
| 515 | 5-Me | H | B3 | COOH | $CH_2$ | J15 | S |
| 516 | 5-Et | H | B3 | COOH | $CH_2$ | J15 | S |
| 517 | 5-F | H | B3 | COOH | $CH_2$ | J15 | S |
| 518 | 5-Cl | H | B3 | COOH | $CH_2$ | J15 | S |
| 519 | 5-$CF_3$ | H | B3 | COOH | $CH_2$ | J15 | S |
| 520 | 5-MeO | H | B3 | COOH | $CH_2$ | J15 | S |
| 521 | 5-EtO | H | B3 | COOH | $CH_2$ | J15 | S |
| 522 | 5-Me | 6-Me | B3 | COOH | $CH_2$ | J15 | S |
| 523 | 5-Et | 6-Et | B3 | COOH | $CH_2$ | J15 | S |
| 524 | 5-F | 6-F | B3 | COOH | $CH_2$ | J15 | S |
| 525 | 5-Cl | 6-Cl | B3 | COOH | $CH_2$ | J15 | S |
| 526 | 5-$CF_3$ | 6-$CF_3$ | B3 | COOH | $CH_2$ | J15 | S |

(continued)

| Compound No. | $X^1$ | $X^2$ | B | E | G | J | M |
|---|---|---|---|---|---|---|---|
| 527 | 5-MeO | 6-MeO | B3 | COOH | $CH_2$ | J15 | S |
| 528 | 5-EtO | 6-EtO | B3 | COOH | $CH_2$ | J15 | S |
| 529 | H | H | B3 | COOH | $CH_2$ | J28 | S |
| 530 | 5-Me | H | B3 | COOH | $CH_2$ | J28 | S |
| 531 | 5-Et | H | B3 | COOH | $CH_2$ | J28 | S |
| 532 | 5-F | H | B3 | COOH | $CH_2$ | J28 | S |
| 533 | 5-Cl | H | B3 | COOH | $CH_2$ | J28 | S |
| 534 | 5-$CF_3$ | H | B3 | COOH | $CH_2$ | J28 | S |
| 535 | 5-MeO | H | B3 | COOH | $CH_2$ | J28 | S |
| 536 | 5-EtO | H | B3 | COOH | $CH_2$ | J28 | S |
| 537 | 5-Me | 6-Me | B3 | COOH | $CH_2$ | J28 | S |
| 538 | 5-Et | 6-Et | B3 | COOH | $CH_2$ | J28 | S |
| 539 | 5-F | 6-F | B3 | COOH | $CH_2$ | J28 | S |
| 540 | 5-Cl | 6-Cl | B3 | COOH | $CH_2$ | J28 | S |
| 541 | 5-$CF_3$ | 6-$CF_3$ | B3 | COOH | $CH_2$ | J28 | S |
| 542 | 5-MeO | 6-MeO | B3 | COOH | $CH_2$ | J28 | S |
| 543 | 5-EtO | 6-EtO | B3 | COOH | $CH_2$ | J28 | S |
| 544 | H | H | B3 | COOH | $CH_2$ | J35 | S |
| 545 | 5-Me | H | B3 | COOH | $CH_2$ | J35 | S |
| 546 | 5-Et | H | B3 | COOH | $CH_2$ | J35 | S |
| 547 | 5-F | H | B3 | COOH | $CH_2$ | J35 | S |
| 548 | 5-Cl | H | B3 | COOH | $CH_2$ | J35 | S |
| 549 | 5-$CF_3$ | H | B3 | COOH | $CH_2$ | J35 | S |
| 550 | 5-MeO | H | B3 | COOH | $CH_2$ | J35 | S |
| 551 | 5-EtO | H | B3 | COOH | $CH_2$ | J35 | S |
| 552 | 5-Me | 6-Me | B3 | COOH | $CH_2$ | J35 | S |
| 553 | 5-Et | 6-Et | B3 | COOH | $CH_2$ | J35 | S |
| 554 | 5-F | 6-F | B3 | COOH | $CH_2$ | J35 | S |
| 555 | 5-Cl | 6-Cl | B3 | COOH | $CH_2$ | J35 | S |
| 556 | 5-$CF_3$ | 6-$CF_3$ | B3 | COOH | $CH_2$ | J35 | S |
| 557 | 5-MeO | 6-MeO | B3 | COOH | $CH_2$ | J35 | S |
| 558 | 5-EtO | 6-EtO | B3 | COOH | $CH_2$ | J35 | S |
| 559 | H | H | B3 | COOH | $CH_2$ | J37 | S |
| 560 | 5-Me | H | B3 | COOH | $CH_2$ | J37 | S |
| 561 | 5-Et | H | B3 | COOH | $CH_2$ | J37 | S |
| 562 | 5-F | H | B3 | COOH | $CH_2$ | J37 | S |
| 563 | 5-Cl | H | B3 | COOH | $CH_2$ | J37 | S |
| 564 | 5-$CF_3$ | H | B3 | COOH | $CH_2$ | J37 | S |

(continued)

| Compound No. | $X^1$ | $X^2$ | B | E | G | J | M |
|---|---|---|---|---|---|---|---|
| 565 | 5-MeO | H | B3 | COOH | $CH_2$ | J37 | S |
| 566 | 5-EtO | H | B3 | COOH | $CH_2$ | J37 | S |
| 567 | 5-Me | 6-Me | B3 | COOH | $CH_2$ | J37 | S |
| 568 | 5-Et | 6-Et | B3 | COOH | $CH_2$ | J37 | S |
| 569 | 5-F | 6-F | B3 | COOH | $CH_2$ | J37 | S |
| 570 | 5-Cl | 6-Cl | B3 | COOH | $CH_2$ | J37 | S |
| 571 | 5-$CF_3$ | 6-$CF_3$ | B3 | COOH | $CH_2$ | J37 | S |
| 572 | 5-MeO | 6-MeO | B3 | COOH | $CH_2$ | J37 | S |
| 573 | 5-EtO | 6-EtO | B3 | COOH | $CH_2$ | J37 | S |
| 574 | H | H | B3 | COOH | $CH_2$ | J39 | S |
| 575 | 5-Me | H | B3 | COOH | $CH_2$ | J39 | S |
| 576 | 5-Et | H | B3 | COOH | $CH_2$ | J39 | S |
| 577 | 5-F | H | B3 | COOH | $CH_2$ | J39 | S |
| 578 | 5-Cl | H | B3 | COOH | $CH_2$ | J39 | S |
| 579 | 5-$CF_3$ | H | B3 | COOH | $CH_2$ | J39 | S |
| 580 | 5-MeO | H | B3 | COOH | $CH_2$ | J39 | S |
| 581 | 5-EtO | H | B3 | COOH | $CH_2$ | J39 | S |
| 582 | 5-Me | 6-Me | B3 | COOH | $CH_2$ | J39 | S |
| 583 | 5-Et | 6-Et | B3 | COOH | $CH_2$ | J39 | S |
| 584 | 5-F | 6-F | B3 | COOH | $CH_2$ | J39 | S |
| 585 | 5-Cl | 6-Cl | B3 | COOH | $CH_2$ | J39 | S |
| 586 | 5-$CF_3$ | 6-$CF_3$ | B3 | COOH | $CH_2$ | J39 | S |
| 587 | 5-MeO | 6-MeO | B3 | COOH | $CH_2$ | J39 | S |
| 588 | 5-EtO | 6-EtO | B3 | COOH | $CH_2$ | J39 | S |
| 589 | H | H | B3 | COOH | $CH_2$ | J41 | S |
| 590 | 5-Me | H | B3 | COOH | $CH_2$ | J41 | S |
| 591 | 5-Et | H | B3 | COOH | $CH_2$ | J41 | S |
| 592 | 5-F | H | B3 | COOH | $CH_2$ | J41 | S |
| 593 | 5-Cl | H | B3 | COOH | $CH_2$ | J41 | S |
| 594 | 5-$CF_3$ | H | B3 | COOH | $CH_2$ | J41 | S |
| 595 | 5-MeO | H | B3 | COOH | $CH_2$ | J41 | S |
| 596 | 5-EtO | H | B3 | COOH | $CH_2$ | J41 | S |
| 597 | 5-Me | 6-Me | B3 | COOH | $CH_2$ | J41 | S |
| 598 | 5-Et | 6-Et | B3 | COOH | $CH_2$ | J41 | S |
| 599 | 5-F | 6-F | B3 | COOH | $CH_2$ | J41 | S |
| 600 | 5-Cl | 6-Cl | B3 | COOH | $CH_2$ | J41 | S |
| 601 | 5-$CF_3$ | 6-$CF_3$ | B3 | COOH | $CH_2$ | J41 | S |
| 602 | 5-MeO | 6-MeO | B3 | COOH | $CH_2$ | J41 | S |

(continued)

| Compound No. | $X^1$ | $X^2$ | B | E | G | J | M |
|---|---|---|---|---|---|---|---|
| 603 | 5-EtO | 6-EtO | B3 | COOH | $CH_2$ | J41 | S |
| 604 | H | H | B3 | COOH | $CH_2$ | J45 | S |
| 605 | 5-Me | H | B3 | COOH | $CH_2$ | J45 | S |
| 606 | 5-Et | H | B3 | COOH | $CH_2$ | J45 | S |
| 607 | 5-F | H | B3 | COOH | $CH_2$ | J45 | S |
| 608 | 5-Cl | H | B3 | COOH | $CH_2$ | J45 | S |
| 609 | 5-$CF_3$ | H | B3 | COOH | $CH_2$ | J45 | S |
| 610 | 5-MeO | H | B3 | COOH | $CH_2$ | J45 | S |
| 611 | 5-EtO | H | B3 | COOH | $CH_2$ | J45 | S |
| 612 | 5-Me | 6-Me | B3 | COOH | $CH_2$ | J45 | S |
| 613 | 5-Et | 6-Et | B3 | COOH | $CH_2$ | J45 | S |
| 614 | 5-F | 6-F | B3 | COOH | $CH_2$ | J45 | S |
| 615 | 5-Cl | 6-Cl | B3 | COOH | $CH_2$ | J45 | S |
| 616 | 5-$CF_3$ | 6-$CF_3$ | B3 | COOH | $CH_2$ | J45 | S |
| 617 | 5-MeO | 6-MeO | B3 | COOH | $CH_2$ | J45 | S |
| 618 | 5-EtO | 6-EtO | B3 | COOH | $CH_2$ | J45 | S |
| 619 | H | H | B3 | COOH | $CH_2$ | J47 | S |
| 620 | 5-Me | H | B3 | COOH | $CH_2$ | J47 | S |
| 621 | 5-Et | H | B3 | COOH | $CH_2$ | J47 | S |
| 622 | 5-F | H | B3 | COOH | $CH_2$ | J47 | S |
| 623 | 5-Cl | H | B3 | COOH | $CH_2$ | J47 | S |
| 624 | 5-$CF_3$ | H | B3 | COOH | $CH_2$ | J47 | S |
| 625 | 5-MeO | H | B3 | COOH | $CH_2$ | J47 | S |
| 626 | 5-EtO | H | B3 | COOH | $CH_2$ | J47 | S |
| 627 | 5-Me | 6-Me | B3 | COOH | $CH_2$ | J47 | S |
| 628 | 5-Et | 6-Et | B3 | COOH | $CH_2$ | J47 | S |
| 629 | 5-F | 6-F | B3 | COOH | $CH_2$ | J47 | S |
| 630 | 5-Cl | 6-Cl | B3 | COOH | $CH_2$ | J47 | S |
| 631 | 5-$CF_3$ | 6-$CF_3$ | B3 | COOH | $CH_2$ | J47 | S |
| 632 | 5-MeO | 6-MeO | B3 | COOH | $CH_2$ | J47 | S |
| 633 | 5-EtO | 6-EtO | B3 | COOH | $CH_2$ | J47 | S |
| 634 | 5-EtO | H | B3 | COOH | $CH_2$ | J47 | S |
| 635 | 5-Me | 6-Me | B3 | COOH | $CH_2$ | J47 | S |
| 636 | 5-Et | 6-Et | B3 | COOH | $CH_2$ | J47 | S |
| 637 | 5-F | 6-F | B3 | COOH | $CH_2$ | J47 | S |
| 638 | 5-Cl | 6-Cl | B3 | COOH | $CH_2$ | J47 | S |
| 639 | 5-$CF_3$ | 6-$CF_3$ | B3 | COOH | $CH_2$ | J47 | S |
| 640 | 5-MeO | 6-MeO | B3 | COOH | $CH_2$ | J47 | S |

(continued)

| Compound No. | $X^1$ | $X^2$ | B | E | G | J | M |
|---|---|---|---|---|---|---|---|
| 641 | 5-EtO | 6-EtO | B3 | COOH | $CH_2$ | J47 | S |
| 642 | H | H | B3 | COOH | $CH_2$ | J50 | S |
| 643 | 5-Me | H | B3 | COOH | $CH_2$ | J50 | S |
| 644 | 5-Et | H | B3 | COOH | $CH_2$ | J50 | S |
| 645 | 5-F | H | B3 | COOH | $CH_2$ | J50 | S |
| 646 | 5-Cl | H | B3 | COOH | $CH_2$ | J50 | S |
| 647 | 5-$CF_3$ | H | B3 | COOH | $CH_2$ | J50 | S |
| 648 | 5-MeO | H | B3 | COOH | $CH_2$ | J50 | S |
| 649 | 5-EtO | H | B3 | COOH | $CH_2$ | J50 | S |
| 650 | 5-Me | 6-Me | B3 | COOH | $CH_2$ | J50 | S |
| 651 | 5-Et | 6-Et | B3 | COOH | $CH_2$ | J50 | S |
| 652 | 5-F | 6-F | B3 | COOH | $CH_2$ | J50 | S |
| 653 | 5-Cl | 6-Cl | B3 | COOH | $CH_2$ | J50 | S |
| 654 | 5-$CF_3$ | 6-$CF_3$ | B3 | COOH | $CH_2$ | J50 | S |
| 655 | 5-MeO | 6-MeO | B3 | COOH | $CH_2$ | J50 | S |
| 656 | 5-EtO | 6-EtO | B3 | COOH | $CH_2$ | J50 | S |
| 657 | 5-Me | H | B3 | COOH | $CH_2$ | J54 | S |
| 658 | 5-Et | H | B3 | COOH | $CH_2$ | J54 | S |
| 659 | 5-F | H | B3 | COOH | $CH_2$ | J54 | S |
| 660 | 5-Cl | H | B3 | COOH | $CH_2$ | J54 | S |
| 661 | 5-$CF_3$ | H | B3 | COOH | $CH_2$ | J54 | S |
| 662 | 5-MeO | H | B3 | COOH | $CH_2$ | J54 | S |
| 663 | 5-EtO | H | B3 | COOH | $CH_2$ | J54 | S |
| 664 | 5-Me | 6-Me | B3 | COOH | $CH_2$ | J54 | S |
| 665 | 5-Et | 6-Et | B3 | COOH | $CH_2$ | J54 | S |
| 666 | 5-F | 6-F | B3 | COOH | $CH_2$ | J54 | S |
| 667 | 5-Cl | 6-Cl | B3 | COOH | $CH_2$ | J54 | S |
| 668 | 5-$CF_3$ | 6-$CF_3$ | B3 | COOH | $CH_2$ | J54 | S |
| 669 | 5-MeO | 6-MeO | B3 | COOH | $CH_2$ | J54 | S |
| 670 | 5-EtO | 6-EtO | B3 | COOH | $CH_2$ | J54 | S |
| 671 | H | H | B3 | COOH | $CH_2$ | J62 | S |
| 672 | 5-Me | H | B3 | COOH | $CH_2$ | J62 | S |
| 673 | 5-Et | H | B3 | COOH | $CH_2$ | J62 | S |
| 674 | 5-F | H | B3 | COOH | $CH_2$ | J62 | S |
| 675 | 5-Cl | H | B3 | COOH | $CH_2$ | J62 | S |
| 676 | 5-$CF_3$ | H | B3 | COOH | $CH_2$ | J62 | S |
| 677 | 5-MeO | H | B3 | COOH | $CH_2$ | J62 | S |
| 678 | 5-EtO | H | B3 | COOH | $CH_2$ | J62 | S |

(continued)

| Compound No. | $X^1$ | $X^2$ | B | E | G | J | M |
|---|---|---|---|---|---|---|---|
| 679 | 5-Me | 6-Me | B3 | COOH | $CH_2$ | J62 | S |
| 680 | 5-Et | 6-Et | B3 | COOH | $CH_2$ | J62 | S |
| 681 | 5-F | 6-F | B3 | COOH | $CH_2$ | J62 | S |
| 682 | 5-Cl | 6-Cl | B3 | COOH | $CH_2$ | J62 | S |
| 683 | 5-$CF_3$ | 6-$CF_3$ | B3 | COOH | $CH_2$ | J62 | S |
| 684 | 5-MeO | 6-MeO | B3 | COOH | $CH_2$ | J62 | S |
| 685 | 5-EtO | 6-EtO | B3 | COOH | $CH_2$ | J62 | S |
| 686 | 5-Me | H | B3 | COOH | $CH_2$ | J64 | S |
| 687 | 5-Et | H | B3 | COOH | $CH_2$ | J64 | S |
| 688 | 5-F | H | B3 | COOH | $CH_2$ | J64 | S |
| 689 | 5-Cl | H | B3 | COOH | $CH_2$ | J64 | S |
| 690 | 5-$CF_3$ | H | B3 | COOH | $CH_2$ | J64 | S |
| 691 | 5-MeO | H | B3 | COOH | $CH_2$ | J64 | S |
| 692 | 5-EtO | H | B3 | COOH | $CH_2$ | J64 | S |
| 693 | 5-Et | 6-Et | B3 | COOH | $CH_2$ | J64 | S |
| 694 | 5-F | 6-F | B3 | COOH | $CH_2$ | J64 | S |
| 695 | 5-Cl | 6-Cl | B3 | COOH | $CH_2$ | J64 | S |
| 696 | 5-$CF_3$ | 6-$CF_3$ | B3 | COOH | $CH_2$ | J64 | S |
| 697 | 5-MeO | 6-MeO | B3 | COOH | $CH_2$ | J64 | S |
| 698 | 5-EtO | 6-EtO | B3 | COOH | $CH_2$ | J64 | S |
| 699 | H | H | B3 | COOH | $CH_2$ | J65 | S |
| 700 | 5-Me | H | B3 | COOH | $CH_2$ | J65 | S |
| 701 | 5-Et | H | B3 | COOH | $CH_2$ | J65 | S |
| 702 | 5-F | H | B3 | COOH | $CH_2$ | J65 | S |
| 703 | 5-Cl | H | B3 | COOH | $CH_2$ | J65 | S |
| 704 | 5-$CF_3$ | H | B3 | COOH | $CH_2$ | J65 | S |
| 705 | 5-MeO | H | B3 | COOH | $CH_2$ | J65 | S |
| 706 | 5-EtO | H | B3 | COOH | $CH_2$ | J65 | S |
| 707 | 5-Me | 6-Me | B3 | COOH | $CH_2$ | J65 | S |
| 708 | 5-Et | 6-Et | B3 | COOH | $CH_2$ | J65 | S |
| 709 | 5-F | 6-F | B3 | COOH | $CH_2$ | J65 | S |
| 710 | 5-Cl | 6-Cl | B3 | COOH | $CH_2$ | J65 | S |
| 711 | 5-$CF_3$ | 6-$CF_3$ | B3 | COOH | $CH_2$ | J65 | S |
| 712 | 5-MeO | 6-MeO | B3 | COOH | $CH_2$ | J65 | S |
| 713 | 5-EtO | 6-EtO | B3 | COOH | $CH_2$ | J65 | S |
| 714 | H | H | B3 | COOH | $CH_2$ | J66 | S |
| 715 | 5-Me | H | B3 | COOH | $CH_2$ | J66 | S |
| 716 | 5-Et | H | B3 | COOH | $CH_2$ | J66 | S |

(continued)

| Compound No. | $X^1$ | $X^2$ | B | E | G | J | M |
|---|---|---|---|---|---|---|---|
| 717 | 5-F | H | B3 | COOH | $CH_2$ | J66 | S |
| 718 | 5-Cl | H | B3 | COOH | $CH_2$ | J66 | S |
| 719 | 5-$CF_3$ | H | B3 | COOH | $CH_2$ | J66 | S |
| 720 | 5-MeO | H | B3 | COOH | $CH_2$ | J66 | S |
| 721 | 5-EtO | H | B3 | COOH | $CH_2$ | J66 | S |
| 722 | 5-Me | 6-Me | B3 | COOH | $CH_2$ | J66 | S |
| 723 | 5-Et | 6-Et | B3 | COOH | $CH_2$ | J66 | S |
| 724 | 5-F | 6-F | B3 | COOH | $CH_2$ | J66 | S |
| 725 | 5-Cl | 6-Cl | B3 | COOH | $CH_2$ | J66 | S |
| 726 | 5-$CF_3$ | 6-$CF_3$ | B3 | COOH | $CH_2$ | J66 | S |
| 727 | 5-MeO | 6-MeO | B3 | COOH | $CH_2$ | J66 | S |
| 728 | 5-EtO | 6-EtO | B3 | COOH | $CH_2$ | J66 | S |
| 729 | H | H | B3 | COOH | $CH_2$ | J72 | S |
| 730 | 5-Me | H | B3 | COOH | $CH_2$ | J72 | S |
| 731 | 5-Et | H | B3 | COOH | $CH_2$ | J72 | S |
| 732 | 5-F | H | B3 | GOOH | $CH_2$ | J72 | S |
| 733 | 5-Cl | H | B3 | COOH | $CH_2$ | J72 | S |
| 734 | 5-$CF_3$ | H | B3 | COOH | $CH_2$ | J72 | S |
| 735 | 5-MeO | H | B3 | COOH | $CH_2$ | J72 | S |
| 736 | 5-EtO | H | B3 | COOH | $CH_2$ | J72 | S |
| 737 | 5-Me | 6-Me | B3 | COOH | $CH_2$ | J72 | S |
| 738 | 5-Et | 6-Et | B3 | COOH | $CH_2$ | J72 | S |
| 739 | 5-F | 6-F | B3 | COOH | $CH_2$ | J72 | S |
| 740 | 5-Cl | 6-Cl | B3 | COOH | $CH_2$ | J72 | S |
| 741 | 5-$CF_3$ | 6-$CF_3$ | B3 | COOH | $CH_2$ | J72 | S |
| 742 | 5-MeO | 6-MeO | B3 | COOH | $CH_2$ | J72 | S |
| 743 | 5-EtQ | 6-EtO | B3 | COOH | $CH_2$ | J72 | S |
| 744 | H | H | B3 | COOH | $CH_2$ | J73 | S |
| 745 | 5-Me | H | B3 | COOH | $CH_2$ | J73 | S |
| 746 | 5-Et | H | B3 | COOH | $CH_2$ | J73 | S |
| 747 | 5-F | H | B3 | COOH | $CH_2$ | J73 | S |
| 748 | 5-Cl | H | B3 | COOH | $CH_2$ | J73 | S |
| 749 | 5-$CF_3$ | H | B3 | COOH | $CH_2$ | J73 | S |
| 750 | 5-MeO | H | B3 | COOH | $CH_2$ | J73 | S |
| 751 | 5-EtO | H | B3 | COOH | $CH_2$ | J73 | S |
| 752 | 5-Me | 6-Me | B3 | COOH | $CH_2$ | J73 | S |
| 753 | 5-Et | 6-Et | B3 | COOH | $CH_2$ | J73 | S |
| 754 | 5-F | 6-F | B3 | COOH | $CH_2$ | J73 | S |

(continued)

| Compound No. | $X^1$ | $X^2$ | B | E | G | J | M |
|---|---|---|---|---|---|---|---|
| 755 | 5-Cl | 6-Cl | B3 | COOH | $CH_2$ | J73 | S |
| 756 | 5-$CF_3$ | 6-$CF_3$ | B3 | COOH | $CH_2$ | J73 | S |
| 757 | 5-MeO | 6-MeO | B3 | COOH | $CH_2$ | J73 | S |
| 758 | 5-EtO | 6-EtO | B3 | COOH | $CH_2$ | J73 | S |
| 759 | H | H | B3 | COOH | $CH_2$ | J74 | S |
| 760 | 5-Me | H | B3 | COOH | $CH_2$ | J74 | S |
| 761 | 5-Et | H | B3 | COOH | $CH_2$ | J74 | S |
| 762 | 5-F | H | B3 | COOH | $CH_2$ | J74 | S |
| 763 | 5-Cl | H | B3 | COOH | $CH_2$ | J74 | S |
| 764 | 5-$CF_3$ | H | B3 | COOH | $CH_2$ | J74 | S |
| 765 | 5-MeO | H | B3 | COOH | $CH_2$ | J74 | S |
| 766 | 5-EtO | H | B3 | COOH | $CH_2$ | J74 | S |
| 767 | 5-Me | 6-Me | B3 | COOH | $CH_2$ | J74 | S |
| 768 | 5-Et | 6-Et | B3 | COOH | $CH_2$ | J74 | S |
| 769 | 5-F | 6-F | B3 | COOH | $CH_2$ | J74 | S |
| 770 | 5-Cl | 6-Cl | B3 | COOH | $CH_2$ | J74 | S |
| 771 | 5-$CF_3$ | 6-$CF_3$ | B3 | COOH | $CH_2$ | J74 | S |
| 772 | 5-MeO | 6-MeO | B3 | COOH | $CH_2$ | J74 | S |
| 772 | 5-EtO | 6-EtO | B3 | COOH | $CH_2$ | J74 | S |

In benzimidazole derivatives (1) used in the present invention (in some case, this is described as "of the present invention"), in the case where E is COOH and M is S, a derivative can be manufactured, for example, by the following synthetic process (A).

Synthetic process (A)

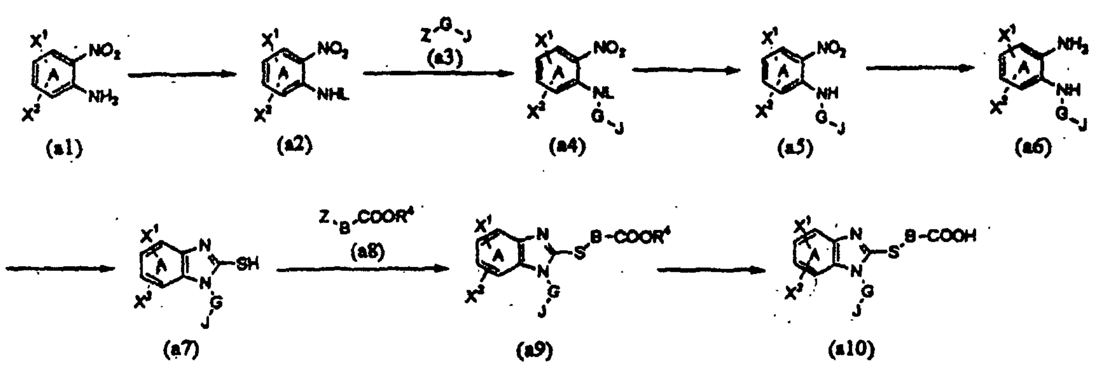


[in the formula, Z is halogen, and the ring marked with A, and B, $X^1$, $X^2$, $R^4$, G and J are defined in the same manner as mentioned above].

Here, the case where the ring marked with A is a benzene ring is explained; however, the processes can be considered to be same also in the case where it is a pyridine ring.

That is, the amino group of a 2-nitroaniline derivative (a1) is protected with L to give (a2). This compound is

made to react with a halide derivative (a3) to give (a4), and this compound is deprotected to give (a5). The nitro group of (a5) is subjected to a reductive reaction to give an orthophenylenediamine derivative (a6). The derivative is treated with $CS_2$ or potassium ethylxanthate to yield a compound (a7), and subsequently this compound is made to react with a halide ester derivative (a8) to afford a compound (a9) of the present invention. If necessary, the $-COOR^4$ of the compound (a9) is hydrolyzed, so that one can obtain a benzimidazole derivative (a10) of the present invention in which $R^4$ is a hydrogen atom. In addition, it is possible directly to obtain the compound (a5) by reacting the halide derivative (a3) with the 2-nitroaniline derivative (a1) as it is not protected. Examples of the protecting group L'include a trifluoroacetyl group, an acetyl group, a t-butoxycarbonyl group, a benzyl group and the like.

The reaction of a 2-nitroaniline derivative (a2) with a halide derivative (a3) can be carried out according to the reaction conditions of a common N-alkylation or N-acylation, for example, in the presence of NaH, $Et_3N$, NaOH, $K_2CO_3$ or the like by stirring at an appropriate temperature from 0°C to 200°C.

The reductive reaction of the nitro group can be carried out according to the common reaction conditions of catalytic reduction, for example, by allowing the nitro group to react with hydrogen gas in the presence of a catalyst such as Pd-C or the like at a temperature from room temperature to 100°C under an acidic, neutral or basic condition. In addition, the reaction can be carried out according to a method in which the nitro group is treated with zinc or tin under an acidic condition, or a method in which the nitro group is treated with zinc powder under a neutral or basic condition.

The cyclization of the orthophenylenediamine derivative (a6) can be carried out by using $CS_2$ or potassium ethylxanthate. The reaction using $CS_2$ can be performed, for example, according to the method (in a pyridine solution) described in J. Org. Chem., 631-637, Vol. 19 (1954) or the method (in an ethanol solution) described in J. Med. Chem., 1175-1187, Vol. 36 (1993). The reaction using potassium ethylxanthate can be performed according to the method described in Organic Synthesis, 569-570, Vol. 4 (1963).

The reaction of a thiobenzimidazole (a7) with a halide ester (a8) can be carried out according to conditions of a common S-alkylatibn reaction, for example, in the presence of a base such as NaH, $Et_3N$, NaOH, $K_2CO_3$ or the like by stirring at a temperature from 0°C to 200°C.

The elimination reaction of a carboxy protection group $R^4$ is preferably carried out by using a method in which hydrolysis is performed with an alkali such as lithium hydroxide or an acid such as hydrochloric acid or trifluoroacetic acid.

There is no special limitation on the synthesis of the abovementioned halide derivative; however, the halide derivative can be synthesized, for example, according to halogenation of a hydroxyl group, a radical halogenation reaction of a methyl group or the like which is generally used in organic syntheses.

In benzimidazole derivatives (1) of the present invention, in the case where E is COOH, M is S, and G is an amide bond, a derivative can be manufactured by the following synthetic process (B).

Synthetic process (B)

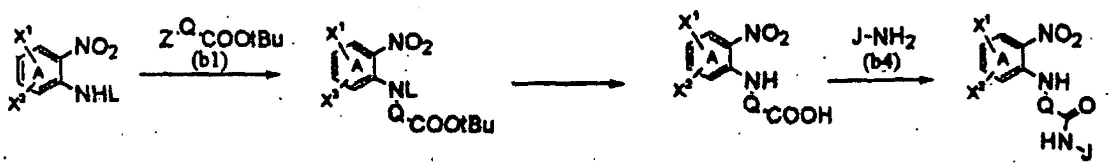


(a2) (b2) (b3) (b5)

[in the formula, Q is a methylene group, a phenylene group or the like; Z is a halogen atom; the ring marked with A, and $X^1$, $X^2$, J and L are defined in the same manner as mentioned above].

Here, the processes are explained in the case where the ring marked with A is a benzene ring; however, the processes may be considered to be same also in the case where it is a pyridine ring.

That is, an aniline derivative (a2) is made to react with a tert-butyl ester halide (b1) to give a compound (b2), and this compound is hydrolyzed under an acidic condition to give (b3). The obtained product (b3) is allowed to condense with an amine derivative (b4) to give (b5). Subsequently, the reductive reaction, the cyclization, the alkylation and the deprotection are carried out in the same manner as in the synthetic process (A), and a benzimidazole derivative of the present invention can be obtained.

The condensation amidation is carried out under the conditions of a common process using a condensing agent. Examples of the condensing agent are DCC, DIPC, EDC=WSCI, WSCI·HCl, BOP, DPPA and the like. The condensing agent is used singly or in combination with HONSu, HOBt, HOOBt or the like. The reaction is carried out

in an appropriate solvent such as THF, chloroform, t-butanol or the like at an appropriate temperature from 0°C to 200°C.

In thiobenzimidazole derivatives (1) of the present invention, in the case where E is COOH, M is S, and G is an ether bond, a derivative can be manufactured by the following synthetic process (C).

Synthetic process (C)

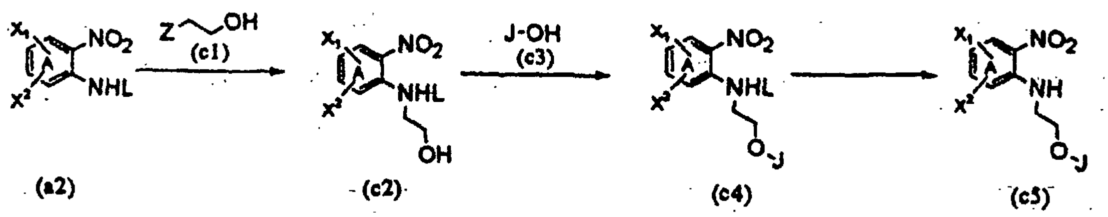


(a2) (c2) (c4) (c5)

[in the formula, Z is halogen; the ring marked with A, and $X^1$, $X^2$, J and L are defined in the same manner as mentioned above].

Here, the processes are explained in the case where the ring marked with A is a benzene ring; however, the processes may be considered to be same also in the case where it is a pyridine ring.

That is, an aniline derivative (a2) is made to react, for example, with a haloalcohol derivative (c1) to give a compound (c2). The compound (c2) is allowed to react with a phenol derivative (c3) to give an ethereal body (c4), and the compound (c4) is hydrolyzed to give (c5). Subsequently, the reductive reaction, the cyclization, the alkylation and the deprotection are carried out in the same manner as in the synthetic process (A), and a benzimidazole derivative of the present invention can be obtained.

The etherification is carried out by using a phosphine compound such as triphenylphosphine or tributylphosphine, and an azo compound such as DEAD or TMAD in an appropriate solvent such as N-methylmorpholine or THF at an appropriate temperature from 0°C to 200°C by Mitsunobu Reaction or its analogous reaction. Other reactions can be carried out in the same manners as in the synthetic process (A).

In benzimidazole derivatives (1) of the present invention, in the case where M is sulfoxide or sulfone, a derivative can be manufactured by the following synthetic process (D).

Synthetic process (D)

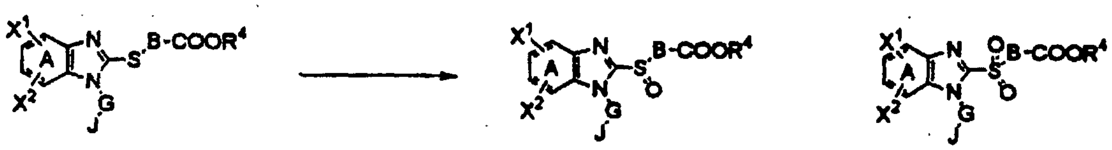

(a9) (d1) (d2)

[in the formula, the ring marked with A, and B, $X^1$, $X^2$, J and $R^4$ are defined in the same manner as mentioned above].

That is, a thiobenzimidazole compound (a9) is made to react with a peroxide compound in an appropriate solvent to give a sulfoxide derivative (d1) and/or a sulfone derivative (d2). The peroxide compound to be used is, for example, perbenzoic acid, m-chloroperbenzoic acid, peracetic acid, hydrogen peroxide or the like; and the solvent to be used is, for example, chloroform, dichloromethane or the like. The using ratio of the compound (a9) to the peroxide compound is not specifically limited, and it can be appropriately selected from a wide range; however, generally speaking, they are used preferably at about 1.2 to 5 times molar ratio. Each reaction is carried out commonly at a temperature from 0°C to 50°C, preferably from 0°C to room temperature, and the reaction finishes generally at about 4 to 20 hr.

In benzimidazole derivatives (1) of the present invention, in the case where M is a single bond, a derivative can be manufactured by the following synthetic process (E).

Synthetic process (E)

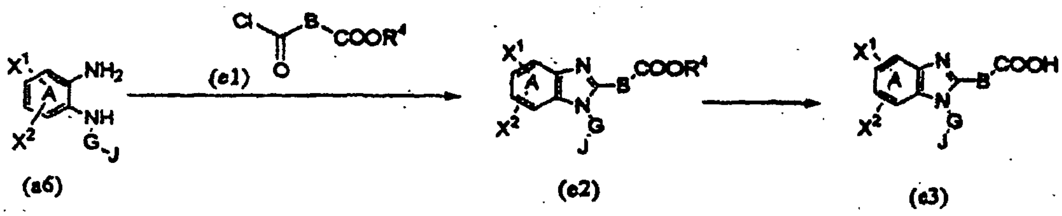

[in the formula, the ring marked with A, and B, $X^1$, $X^2$, G, J and $R^4$ are defined in the same manner as mentioned above].

That is, a benzimidazole derivative (e2) can be obtained by reacting an orthophenylenediamine derivative (a6) with a known acid chloride derivative (e1). A benzimidazole derivative (e3) of the present invention can be obtained by hydrolyzing the $-COOR^4$ moiety of the compound (e2) at need.

In addition, a benzimidazole derivative of the present invention can be synthesized referring to the method of J. Med. Chem., 1175-1187, Vol. 36 (1993), US 5,124,336, US 5,021,443 or the like.

At need, a benzimidazole derivative of the present invention can be converted to a pharmaceutically permissible nontoxic cationic salt. The salt is formed with an alkali metal ion such as $Na^+$ or $K^+$; an alkaline earth metal ion such as $Mg^+$ or $Ca^+$; a metal ion such as $Al^{3+}$ or $Zn^{2+}$; or an organic base such as ammonia, triethylamine, ethylenediamine, propanediamine, pyrrolidine, piperidine, piperazine, pyridine, lysine, choline, ethanolamine, N,N-dimethylethanolamine, 4-hydroxypiperidine, glucosamine . or N-methylglucamine. $Na^+$, $Ca^{2+}$, lysine, choline, N,N-dimethylethanolamine and N-methylglucamine are especially preferable.

A benzimidazole derivative expressed by the above formula (1) of the present invention has an activity for inhibiting human chymase activity, and can be used as a preventive agent and/or a treating agent which is clinically applicable as human chymase inhibitor.

In addition, a benzimidazole derivative of the present invention can be administered orally or parentally by preparing a pharmaceutical composition with a pharmaceutically permissible carrier and formulating the composition into various dosage forms. The parenteral administrations are, for example, intravenous, subcutaneous, intramuscular, percutaneous, intrarectal and nasal administrations, and instillation.

The dosage forms of the pharmaceutical composition are the following. In the case of an oral administration agent, they are tables, pills, granules, powder, a solution, a suspension, a syrup, capsules and the like.

Here, the tables can be formulated according to a common method by using pharmaceutically permissible carriers such as a filler, a binder and a disintegrator. The pills, granules and powder also can be formulated according to common methods by using a filler and the like in the same manner as in the tablets. The solution, suspension and syrup can be formulated according to common methods by using a glycerin ester, an alcohol, water, a vegetable oil or the like. The capsules can be formulated by filling granules, powder, a solution or the like in a gelatin capsule or the like.

Out of the parenteral administrations, in the cases of the intravenous administration, subcutaneous administration and intramuscular administration, the agent can be administered in a dosage form of an injection. The injection is prepared by dissolving a benzimidazole derivative, for example, in a water-soluble solvent such as physiological saline or by dissolving a benzimidazole derivative, for example, in a water-insoluble solvent consisting of propylene glycol, polyethylene glycol or an organic ester such as a vegetable oil.

In the case of the percutaneous administration, the agent can be used in a dosage form, for example, of an ointment, a cream or the like. The ointment can be formulated by mixing a benzimidazole derivative with a fat and oil, vaseline or the like. The cream can be formulated by mixing a benzimidazole derivative with an emulsifier.

In the case of the intrarectal administration, the agent can be formulated into a suppository by using a gelatin soft capsule or the like.

In the case of the nasal administration, the agent can be used in a dosage form consisting of a liquid or powdery composition. As the base of the liquid agent, water, saline, a phosphate buffer, an acetate buffer or the like is used, and the base may further contain a surfactant, an antioxidant, a stabilizer, a preservative or a thickener. The bases of the powdery agent are, for example, a water-absorbing substance such as an easily water-soluble polyacrylic acid salt, a cellulose lower alkyl ether, polyethylene glycol, polyvinyl pyrrolidone, amylose or pullulan, and, for example, a scarcely water-soluble substance such as a cellulose, a starch, a protein, a gum or a cross-linked vinyl polymer. The water-absorbing substance is preferred as the base. Further, these substances may be used by mixing with each other. Furthermore, to the powdery dosage form, an antioxidant, a coloring agent, a preservative, an antiseptic, a corrigent or the like may be added. Each of the liquid agent and powdery agent can be administered, for example, by using a sprayer or the like.

In the case of instillation, the agent can be used in the form of an aqueous or nonaqueous eye drop. In the aqueous eye drop, as the solvent, sterile purified water, physiological saline or the like can be used. In the case where only the sterile purified water is used as the solvent, the agent can be used in an aqueous suspension eye drop by adding a suspending agent such as a surfactant or a polymeric thickener, and in addition, it can be used in the form of a solubilized eye drop by adding a solubilizer such as a nonionic surfactant. In the nonaqueous eye drop, a nonaqueous solvent for injection can be used as the solvent, and the formed eye drop can be used as a nonaqueous suspension eye drop.

In the case where the agent is administered into an eye by a method other than the eye drops, it is formulated into an ophthalmic ointment, a coating solution, a catapasm, an insert or the like.

Further, in the case where the agent is inhaled through the noses, the mouth or the like, it is inhaled in a dosage form of a solution or a suspension consisting of a benzimidazole derivative and a generally used pharmaceutical vehicle by using, for example, an aerosol sprayer for inhalation or the like. Further, a benzimidazole derivative formed into a dry powdery body can be administered by using an inhalator or the like which allows the powdery body to come into direct contact with the lung.

Into these various pharmaceutical preparations, a pharmaceutically permissible support such as an isotonizing agent, a preservative, an antiseptic, a wetting agent, a buffering agent, an emulsifier, a dispersing agent or a stabilizer can be added at need.

In addition, these various pharmaceutical preparations can be sterilized through a treatment such as the addition of a sterilizing agent, the filtration using a bacteria retention filter, heating or irradiation at need. Or, an aseptic solid preparation is produced, and the preparation is dissolved or suspended in an appropriate aseptic solution directly before the use.

The dose of the benzimidazole derivative of the present invention depends on the kind of the disease, the administration route, the symptom, the age, the sex and the body weight of the patient, and the like; however, in oral administration, it is generally about 1-500 mg/day/person, preferably, 10-300 mg/day/person. In the case of a parenteral administration such as intravenous, subcutaneous, intramuscular, percutaneous, intrarectal or nasal administration, instillation, or inhalation, it is about 0.1-100 mg/day/person, preferably 0.3-30 mg/day/person.

Here, examples of the disease associated with human chymase include inflammatory diseases, allergy diseases, respiratory diseases, cardiovascular diseases, bone/cartridge metabolic diseases and the like.

Further, when an inhibitor against human chymase activity of the present invention is used as a preventing agent, it can be administered according to a known method in advance in accordance with each symptom.

Examples

The present invention will be explained henceforth with examples, while the present invention is not restricted by the examples.

[Example 1]

Production of 4-((1-((1-naphthyl)methyl)-5-methoxybenzimidazole)-2-ylthio)butyric acid (Compound No. 127)

[Process 1]

3,026 mg of 4-methoxy-2-nitroaniline (18 mmol) was dissolved in 60 ml of acetonitrile, and the resulting solution was refluxed for 3 hr after the addition of 3.00 ml of trifluoroacetic anhydride (21.6 mmol). After it was cooled to room temperature, the reaction mixture was concentrated in vacuo, and the residue was crystallized with adding hexane. This was filtered, and the product on the filter was dried to give 1.396 g of 4-methoxy-2-nitrotrifluoroacetanilide (82% yield).

[Process 2]

Subsequently, 1.59 g of 4-methoxy-2-nitrotrifluoroacetanilide (6 mmol) was dissolved in 20 ml of N,N-dimethylformamide, to the solution were added 1.27 g of potassium carbonate (7.2 mmol) and 1.08 g of 1-chloromethylnaphthalene (7.8 mmol), and the mixture was stirred at 100°C for 3 hr. After 3 hr, into the reaction mixture was added 10 ml of 5M sodium hydroxide aqueous solution, and the resulting mixture was heated at 100 °C for 1.5 hr. The reaction mixture was cooled to room temperature, and dissolved in 100 ml of diethyl ether, and the solution was washed with 50 ml of water and dried over magnesium sulfate. The organic layer was concentrated in vacuo, the residue was purified by silica gel column chromatography (hexane : ethyl acetate= 5:1), the objective fraction was concentrated in vacuo, and the residue was dried to yield 1.476 g of orange colored solid of ((1-naphthyl)methyl) (4-methoxy-2-nitrophenyl)amine (80% yield).

[Process 3]

Subsequently, to 1.476 g of ((1-naphthyl)methyl)(4-methoxy-2-nitrophenyl)amine (4.79 mmol) were added 8 ml of 1,4-dioxane and 8 ml of ethanol, and further 0.6 ml of 5M aqueous solution of sodium hydroxide and 1.25 g of zinc powder, and the resulting mixture was heated at 100°C. After 2 hr heating, 0.6 ml of 5M aqueous solution of sodium hydroxide and 1.25 g of zinc powder were further added to the reaction mixture, and heating was continued. After 3 hr from the start of the reaction, the reaction mixture was cooled to room temperature, it was filtered through celite, and the filtrate was concentrated in vacuo. The obtained residue was dissolved in ethyl acetate, and the solution was washed with brine and concentrated in vacuo. The residue was dried to give 1.21 g of brown oily ((1-naphthyl)methyl)(2-amino-4-methoxyphenyl)amine (91% yield).

[Process 4]

Successively, 1.2 g of ((1-naphthyl)methyl)(2-amino-4-methoxyphenyl)amine (4.3 mmol) was dissolved in 30 ml of ethanol, and the solution was refluxed after the addition of 10 ml of carbon disulfide. After 12 hr, the reaction mixture was concentrated in vacuo, 10 ml of ethanol was added to the residue, and the mixture was treated in an ultrasonic bath. The formed solid was filtered, and it was washed with ethanol (2 ml × 2) and dried to yield 620 mg of 1-(1-naphthyl)methyl-5-methoxybenzimidazole-2-thiol (45% yield in two processes).

[Process 5]

To 32 mg of 1-(1-naphthyl)methyl-5-methoxybenzimidazole-2-thiol (0.1 mmol) was added 2 ml of N,N-dimethylformamide, and subsequently 21 μl of triethylamine (0.15 mmol) and then 17 μl of ethyl 4-bromo-n-butyrate (0.12 mmol) were added. The mixture was stirred at 80°C for 10 hr under heating. Subsequently, water was added to the reaction mixture, the resulting mixture was extracted with diethyl ether, and the organic layer was concentrated in vacuo. The residue was purified by preparative thin layer chromatography (hexane : ethyl acetate = 1:1), and the product was collected and dried.

[Process 6]

Successively, the obtained residue was dissolved in 5 ml of methanol, 0.5 ml of 4M aqueous solution of lithium hydroxide was added to the solution, and the mixture was heated at 50°C for 3 hr. After 3 hr, the reaction mixture was neutralized with 6M hydrochloric acid, and the mixture was extracted with chloroform. The organic layer was concentrated in vacuo, and the residue was purified by a preparative thin layer chromatography (hexane-ethyl acetate=1:1). The objective fraction was concentrated, and the residue was dried to yield 13.4 mg of 4-((1-((1-naphthyl)methyl)-5-methoxybenzimidazole)-2-ylthio)butyric acid (33% yield in two processes).
LC-MS : calculated value=406.14, and measured value $(M+1)^+$=407.3

[Example 2]

<u>Production of 4-((1-((1-naphthyl)methyl)-5,6-difluorobenzimidazole)-2-ylthio)butyric acid (Compound No. 131)</u>

500 mg of 3,4-difluoro-5-nitroaniline (2.87 mmol) was dissolved in 5 ml of ethanol, to the solution was added 50 mg of 10% palladium-carbon, and the mixture was heated at 50°C for 12 hr after the atmosphere was substituted with hydrogen. The reaction mixture was cooled and filtered through celite. The filtrate was concentrated in vacuo, and the residue was dried to give 409 mg of 2-amino-4,5-difluoroaniline.

Successively, to the obtained 2-amino-4,5-difluoroaniline as it is were added 5 ml of carbon disulfide and 20 ml of ethanol, and the mixture was heated at 80°C for 12 hr. After cooling, the reaction mixture was concentrated in vacuo, the obtained residue was purified by silica gel column chromatography (hexane ethyl acetate = 1:1), and the product was dried to yield 346 mg of 5,6-difluorobenzimidazole-2-thiol (65% yield in two processes).

Subsequently, 93.1 mg of 5,6-difluorobenzimidazole-2-thiol (0.5 mmol) was dissolved in 2 ml of N.N-dimethylformamide, and the solution was heated at 80°C for 12 hr after the addition of 104 μl of triethylamine and 117 mg of ethyl 4-bromobutyrate. The reaction mixture was cooled, water was added, the mixture was extracted with diethyl ether, and the organic layer was concentrated in vacuo. The residue was purified by preparative thin layer chromatography (hexane-ethyl acetate=1:1), and the product was collected and dried to yield 113 mg of 4-(5,6-difluorobenzimidazole-2-ylthio)butyric acid ethyl ester (75% yield).

Subsequently, 55 mg of 4-(5,6-difluorobenzimidazole-2-ylthio)butyric acid ethyl ester was dissolved in 2 ml of N,N-dimethylformamide, to the solution were added 38 mg of potassium carbonate (0.296 mmol) and 49 mg of 1-chloromethylnaphthalene (0.276 mmol), and the mixture was heated at 80°C for 3 hr. The reaction mixture was cooled to room temperature, and after the addition of water, it was extracted with diethyl ether. The organic layer was dehydrated over magnesium sulfate and concentrated in vacuo, the residue was purified by preparative thin layer chromatography (hexane-ethyl acetate=2:1), and the product was collected and dried to yield 66 mg of 4-(1-((1-naphthyl)methyl)-5,6-difluorobenzimidazole-2-ylthio)butyric acid ethyl ester (82% yield).

Successively, 66 mg of 4-(1-((1-naphthyl)methyl)-5,6-difluorobenzimidazole-2-ylthio)butyric acid ethyl ester was dissolved in 3 ml of methanol, the solution was heated at 50°C for 2 hr after the addition of 0.5 ml of 4M aqueous solution of lithium hydroxide. After 2 hr, the reaction mixture was neutralized with a 6M hydrochloric acid, and the mixture was extracted with chloroform. The organic layer was concentrated in vacuo, the residue was dried to yield 60 mg of 4-(1-((1-naphthyl)methyl)-5,6-difluorobenzimidazole-2-ylthio)butyric acid (98% yield).
LC-MS : calculated value=412.11, and the measured value $(M+1)^+$=413.3

[Example 3]

<u>Production of 4-(1-((1-naphthyl)methyl)-5-cyanobenzimidazole-2-ylthio)butyric acid (Compound No. 363)</u>

[Process 1]

979 mg of 4-cyano-2-nitroaniline (6 mmol) was dissolved in 18 ml of acetonitrile, and the solution was refluxed for 1.5 hr after the addition of 1.00 ml of anhydrous trifluoroacetic acid (7.2 mmol). The reaction mixture was cooled to room temperature and concentrated in vacuo, and the residue was dried to yield 1.396 g of 4-cyano-2-nitrotrifluoroacetanilide (90% yield).

[Process 2]

Subsequently, 3.14 g of 4-cyana-2-nitrotrifluoroacetanilide (12.1 mmol) was dissolved in 30 ml of tetrahydrofuran, 602.3 mg of sodium hydride (60%, oily) (15.1 mmol) was added under ice cooling, and the mixture was stirred at room temperature for 2 hr. Successively, into the reaction mixture was added 3.35 g of 1-bromonaphthalene (15.2 mmol) at room temperature, and the resulting mixture was refluxed. After 10 hr reaction, the reaction mixture was further refluxed for 1.5 hr after the addition of 5 ml of 5M aqueous solution of sodium hydroxide. The reaction mixture was cooled to room temperature and dissolved in 300 ml of ethyl acetate, and the solution was washed with 150 ml of water. The organic layer was dehydrated with magnesium sulfate and concentrated in vacuo. To the residue was added a mixed solvent of hexane-acetone (=2:1), and the formed precipitates were collected by filtration and dried to yield 2.032 g of orange colored solid of ((1-naphthyl)methyl)(4-cyano-2-nitrophenyl)amine (54% yield).
$^1$H-NMR (400MHz, $CDCl_3$)
δ : 5.01 (2 H, d), 6.92 (1 H, d), 7.4 - 7.6 (6 H, m), 7.87 (1 H, dd), 8.55 (1 H, s), 8.74 (1 H, s).
$^{13}$C-NMR (100MHz, $CDCl_3$, ppm)
δ : 45.5, 98.3, 115.4, 118.0, 122.5, 125.62, 125.64, 126.5, 127.1, 129.3, 129.4, 130.8, 131.0, 132.0, 132.3, 134.2, 138.0, 147.1.

[Process 3]

Successively, to 1.60 g of ((1-naphthyl)methyl)(4-cyano-2-nitrophenyl)amine (5.27 mmol) were added 8 ml of ethanol and 8 ml of tetrahydrofuran. Further, 2.9 g of potassium carbonate (21 mmol) was added, the atmosphere was substituted with nitrogen, and subsequently, 160 mg of 10% palladium-carbon was added, and the mixture was heated at 60°C after the atmosphere was substituted with hydrogen. After 2 hr, 160 mg of 10% palladium-carbon was further added, and the mixture was heated at 60°C after the atmosphere was substituted with hydrogen. When 4.5 hr had passed from the start of the reaction, the reaction mixture was cooled to room temperature and filtered through celite, and the filtrate was concentrated in vacuo. The obtained residue was purified by silica gel column chromatography (hexane-acetone=3:1) to yield 1.16 g of colorless liquid of ((1-naphthyl)methyl)(2-amino-4-cyanophenyl)amine.

[Process 4]

Successively, 0.72 g of ((1-naphthyl)methyl)(2-amino-4-cyanophenyl)amine (2.63 mmol) was dissolved in 16 ml of ethanol, and the solution was refluxed after the addition of 507.5 mg of potassium ethylxanthate (3.17 mmol). After 17 hr, 504.2 mg of potassium ethylxanthate (3.15 mmol) was added, and the refluxing was continued. When 40

hr had passed from the start of the reaction, 0.37 g of active Norit A (manufactured by Wako Chemical Co. Ltd.) was added, and the mixture was refluxed for 15 min. The reaction mixture was filtered through celite, 100 ml of hot water of 70°C was added, the mixture was heated at 70°C, and a 40% acetic acid in water was added dropwise. Further, 1 ml of acetic acid was added, and the mixture was cooled to room temperature, and stirred for 1 hr. The formed crystals were collected by filtration and dried to yield 522.6 mg of 1-((1-naphthyl)methyl)-5-cyanobenzimidazole-2-thiol (63% yield in two steps).
$^1$H-NMR (400MHz, $CDCl_3$)
δ: 5.99 (2 H, s), 6.90 (1 H, dd), 7.03 (1 H, d), 7.26 (1 H, dt), 7.36 (1 H, dt), 7.49 - 7.59 (4 H, m), 7.81 (1 H, d), 7.89 (1 H, d), 8.16 (1 H, d).
$^{13}$C-NMR (100MHz, $CDCl_3$, ppm)
δ : 45.6, 105.6, 113.2, 118.7, 122.6, 124.2, 125.0, 125.9, 126.4, 126.5, 128.3, 128.6 129.7, 130.5, 131.3, 133.5, 135.7, 171.9.

[Process 5]

To 65 mg of the obtained 1-((1-naphthyl)methyl)-5-cyanobenzimidazole-2-thiol (0.2 mmol) were added 41 mg of potassium carbonate (0.3 mmol) and 3 ml of N,N-dimethylformamide, and the mixture was heated and stirred at 80°C for 10 hr after the addition of 50 mg of ethyl 4-bromo-n-butyrate (0.26 mmol). Then, the reaction mixture was extracted with diethyl ether after the addition of water and the organic layer was concentrated in vacuo. The obtained residue was purified by a silica gel column chromatography (hexane-ethyl acetate=3:2) to yield 56 mg of a yellow oily substance of 4-(1-((1-naphthyl)methyl)-5-cyanobenzimidazole-2-ylthio)butyric acid ethyl ester (65% yield).
LC/MS: the calculated value M=429.15, and the measured value $(M+1)^+$=430.3.

[Process 6]

56 mg of 4-(1-((1-naphthyl)methyl)-5-cyanobenzimidazole-2-ylthio)butyric acid ethyl ester was dissolved in 5 ml of methanol, and the solution was heated at 50°C for 5 hr after the addition of 0.5 ml of a 4M aqueous solution of lithium hydroxide. After 5 hr, the reaction mixture was extracted with ethyl acetate after the neutralization with a 6M hydrochloric acid. The organic layer was concentrated in vacuo, the residue was purified by preparative thin layer chromatography (chloroform-methanol=20:1), and the product was dried to yield 27 mg of 4-(1-((1-naphthyl)methyl)-5-cyanobenzimidazole-2-ylthio)butyric acid (53% yield)
$^1$H-NMR (400MHz, $CDCl_3$, ppm)
δ: 2:13 (2 H, pentet), 2.45 (2 H, t), 3.50 (2 H, d), 5.85 (2H, s), 6.68 (1H, d), 7.15 (1H, dd), 7.34 (2H, m), 7.61 (2H, m), 7.82 (1H, d), 7.93 (1H, d), 7.97 (1H, s), 8.04 (1H, d).
$^{13}$C-NMR (100MHz, $CDCl_3$, ppm)
δ: 24.3, 31.6, 32.4, 45.4, 104.7, 110.1, 119.6, 121.9, 122.3, 123.1, 125.1, 125.3, 126.1, 126.7, 128.5, 128.8, 129.5, 130.1, 133.4, 155.9, 174.3.

[Example 4]

Production of 4-(1-((1-naphthyl)methyl)-5-cyanobenzimidazole-2-yl)-3,3-dimethylbutyric acid (Compound No. 457)

To 310 mg of ((1-naphthyl)methyl)(4-cyano-2-nitrophenyl)amine (1.13 mmol) was added 6 ml of tetrahydrofuran, the mixture was cooled with ice, and it was stirred at room temperature for 16 hr after the addition of 430 mg of ethyl-(3,3-dimethyl)-glutaryl chloride (2.08 mmol). After 16 hr, the reaction mixture was filtered through celite, the solvent was removed in vacuo, and 6 ml of ethanol and 2 ml of conc. hydrochloric acid were added to the residue, and the mixture was refluxed for 10 hr. The reaction mixture was extracted with ethyl acetate after its pH value was adjusted to 8 with a saturated sodium bicarbonate aqueous solution, and the organic layer was dried over magnesium sulfate. The organic layer was concentrated in vacuo, and the obtained residue was purified by silica gel column chromatography (hexane : acetone = 2:1) to yield 451.26 mg of a yellow oily substance of 4-(1-((1-naphthyl)methyl)-5-cyanobenzimidazole-2-yl)-3,3-dimethylbutyric acid ethyl ester.

Successively, to the obtained compound were added 2 ml of tetrahydrofuran, 4 ml of ethanol and 2 ml of a 2M aqueous solution of sodium hydroxide, and the mixture was stirred at room temperature. After 2 hr, 2 ml of a 2M aqueous solution of sodium hydroxide was further added, and the mixture was heated and stirred at 50°C. After 2 hr, the reaction mixture was cooled and adjusted to pH=3 with a saturated citric acid aqueous solution, and it was extracted with ethyl acetate (40 ml×2) after the addition of 40 ml of water. The organic layer was concentrated in vacuo, the obtained residue was purified by a preparative thin layer chromatograph (Art. 13792 manufactured by Merck Co. Ltd.), and the product was collected to yield 89 mg of 4-(1-naphthyl)methyl)-5-cyanobenzimidazole-2-yl)-3,3-dimethylbutyric

acid (20% yield in 2 steps).
LC/MS: the calculated value M=397.18, and the measured value $(M+1)^+$=398.3

[Example 5]

Production of 4-(1-((1-naphthyl)methyl)benzimidazole-2-ylthio)butyric acid (Compound No. 121)

115.5 g of benzimidazole-2-thiol (0.769 mol) was suspended in 225 ml of toluene, to the suspension were added 102.2 g of triethylamine (1.000 mol, 1.3 eq.) and 175.4 g of ethyl 4-bromobutyrate (0.923 mol, 1.2 eq.), and the resulting mixture was allowed to react at 80°C for 12 hr. After the reaction, the reaction mixture was poured into 500 ml of water, and the mixture was extracted with ethyl acetate of 500 ml and then of 250 ml serially. The combined organic layer was serially washed with 300 ml of water (two times) and with 300 ml of brine, dehydrated with magnesium sulfate (together with 5 g of active carbon) and then concentrated in vacuo to give 196 g of a crude product. The product was recrystalized with 600 ml of diisopropyl ether, the precipitated crystals were washed with cold diisopropyl ether and dried to yield 173 g of ethyl 4-(benzimidazole-2-ylthio)butyrate (85% yield, weakly yellowed white crystal).
Subsequently, 200 ml of N,N-dimethylformamide was added to 20.2 g of ethyl 4-(benzimidazole-2-ylthio)butyrate (76.2 mmol), 16.1 g of 1-chloromethylnaphthalene (91.4 mmol) and 12.6 g of potassium carbonate (91.4 mmol), and the mixture was stirred at 80°C for 5 hr. To the reaction mixture was added 200 ml of water, the resulting mixture was extracted with ethyl acetate (150 ml×3), and the organic layer was washed with water (100 ml×3). The organic layer was dried over magnesium sulfate, the solvent was removed in vacuo, the residue was purified by silica gel chromatography (hexane : ethyl acetate = 9:1) to yield 23.7 g of ethyl 4-(1-((1-naphthyl)methyl)benzimidazole-2-ylthio) butyrate (77% yield).
$^1$H-NMR (270MHz, $CDCl_3$, ppm)
δ: 1.22 (3 H, t, J = 7.09Hz), 2.13 (2 H, m), 2.56 (2 H, t, J = 7.42Hz), 3.48 (2 H, t, 7.09Hz), 4.11 (2 H, q, J = 7.09Hz), 5.80 (2 H, s), 6.68 (1H, d, J = 6.10Hz), 7.05 - 7.11 (2 H, m), 7.20 - 7.31 (2 H, m), 7.57 - 7.65 (2 H, m), 7.72 - 7.80 (2 H, m), 7.93 (1 H, d, J = 7.75Hz), 8.07 (1 H, d, J = 8.08Hz).
Successively, 23.5 g of ethyl 4-(1-((1-naphthyl)methyl)benzimidazole-2-ylthio)butyrate (58.1 mmol) was dissolved in a mixed solvent of 240 ml of THF and 120 ml of methanol, and the solution was stirred at room temperature over night after the addition of 29 ml of a 4M aqueous solution of lithium hydroxide (116 mmol). The reaction mixture was made acidic by adding a saturated citric acid aqueous solution and extracted with ethyl acetate (150 ml×3), and the organic layer was washed with water (150 ml×3) and dried over magnesium sulfate. The solvent was removed with a rotary evaporator. The residue was recrystalized from 900 ml of isopropyl alcohol to yield 20.7 g of 4-(1-((1-naphthyl)methyl)benzimidazole-2-ylthio)butyric acid (95% yield).
The calculated value=376.12, and the measured value $(M+1)^+$=377.2.

[Example 6]

Production of sodium 4-(1-((1-naphthyl)methyl)benzimidazole-2-ylthio)butyrate (Na salt of Compound No. 121)

1.00g of 4-(1-((1-naphthyl)methyl)benzimidazole-2-yothio)butyric acid (2.65 mmol) was suspended in 10 ml of water, and the suspension was stirred at room temperature over night after the addition of 26.5 ml of a 1M aqueous solution of sodium hydroxide (2.65 mmol). The reaction mixture was frozen and lyophilized to yield 981 mg of 4-(1-((1-naphthyl)methyl)benzimidazole-2-ylthio)butyric acid sodium salt (93% yield).
$^1$H-NMR (270MHz, DMSO-$d_6$, ppm)
δ: 1.83 (2 H, m), 1.98 (2 H, m), 3.34 (2 H, m), 5.90 (2 H, s), 6.55 (1 H, d, J = 6.76Hz), 7.08 - 7.19 (2 H, m), 7.27 - 7.38 (2 H, m), 7.61 - 7.64 (3 H, m), 7.84 (1 H, d, J = 8.41Hz), 7.99 (1 H, d, J = 7.75Hz), 8.24 (1 H, d, J = 8.08Hz).

[Example 7]

Production of 4-(1-((8-methyl-1-naphthyl)methyl)benzimidazole-2-ylthio)butyric acid (Compound No. 492)

840 mg of 1,8-dimethylnaphthalene (5.11 mmol) was dissolved in 10 ml of carbon tetrachloride, to the solution were added 890 mg of NBS (5.0 mmol) and 41 mg of AIBN (0.25 mmol), and the mixture was stirred at 90°C for 1 hr. The reaction mixture was filtered to remove the salt, and the filtrate was treated with a rotary evaporator to remove the solvent. The formed residue was purified by silica gel column (hexane) to yield 330 mg of 1-bromomethyl-8-methylnaphthalene (1.4 mmol, 28% yield).
$^1$H-NMR(270MHz, $CDCl_3$, ppm)
δ: 3.124 (3 H, s), 5.170 (2 H,s), 7.361 - 7.412 (3 H,m), 7.535 (1 H, dd), 7.709 - 7.756 (1 H, m), 7.829 (1 H, dd).

Subsequently, to 111 mg of ethyl 4-(benzimidazole-2-ylthio)butyrate (0.42 mmol), 108 mg of 1-bromomethyl-8-methylnaphthalene (0.46 mmol) and 83 mg of potassium carbonate (0.60 mmol) was added 2.0 ml of N,N-dimethylformamide, and the mixture was stirred at 80°C for 3 hr. The reaction mixture was filtered to remove the salt, and the filtrate was treated with a rotary evaporator to remove the solvent. The formed residue was purified by preparative thin layer chromatography (ethyl acetate-chloroform=1:8) to yield 149.8 mg of ethyl 4-(1-((8-methyl-1-naphthyl)methyl) benzimidazole-2-ylthio)butyrate (0.36 mmol, 86% yield).
The calculated value M=418.17, and the measured value $(M+1)^+$=419.1.

Successively, 149.8 mg of ethyl 4-(1-((8-methyl-1-naphthyl)methyl)benzimidazole-2-ylthio)butyrate (0.36 mmol) was dissolved in a mixed solvent of 5 ml of methanol and 5 ml of water, to the solution was added 3 ml of a 1M aqueous solution of sodium hydroxide, and the mixture was stirred at 60°C for 1 hr. The reaction mixture was adjusted at pH=3 with a saturated aqueous solution of citric acid and extracted with ethyl acetate. The organic layer was washed with a small amount of water and dried over magnesium sulfate. The solvent was removed by a rotary evaporator to yield 159.8 mg of 4-(1-((8-methyl-1-naphthyl)methyl) benzimidazole-2-ylthio) butyric acid.
$^1$H-NMR (270MHz, $CDCl_3$, ppm)
δ: 2.158 (2 H, m), 2.596 (2 H, t), 3.066 (3 H, s), 3.454 (2 H, t), 6.086 (2 H, s), 6.471 (1 H, d), 7.048 (1 H, d), 7.129 - 7.204 (2 H, m), 7.242 - 7.298 (1 H, m), 7.389 - 7.435 (2 H, m), 7.751 - 7.793 (3 H, m).
The calculated value M=390.14, and the measured value $(M+1)^+$=391.1

[Example 8]

Production of 4-(1-((8-methyl-1-naphthyl)methyl)-5,6-dimethylbenzimidazole-2-ylthio)-butyric acid (Compound No. 493)

The titled compound was obtained by employing the same procedure described in Example 7, but using ethyl 4-(5,6-dimethylbenzimidazole-2-ylthio)butyrate in the second process.
$^1$H-NMR (270MHz, $CDCl_3$, ppm):
δ: 2.183 (2 H, m), 2.262 (3 H, s), 2.355 (3 H, s), 2.633 (2 H, t), 3.076 (3 H, s), 3.435 (1 H, t), 6.051 (2 H, s), 6.455 (1 H, dd), 6.853 (1 H, d), 7.193 (2 H, t), 7.410 (2 H, d), 7.544 (1 H, s), 7.742 - 7.763 (2 H, m).
The calculated value M=418.17, and the measured value $(M+1)^+$=419.1.

[Example 9]

Production of 5-(1-((1-naphthyl)methyl)benzimidazole-2-ylthio)-4-oxo-3,3-dimethylvaleric acid (Compound No. 494)

5-(1-((1-naphthyl)methyl)benzimidazole-2-ylthio)-4-oxo-3,3-dimethyl-val eric acid was obtained by employing the same procedure described in Example 3, but changing the reagents partially as follows.
[Process 1] 4-cyano-2-nitroaniline → 2-nitroaniline.
[Process 5] ethyl 4-bromo-n-butyrate → ethyl
5-bromo-4-oxo-3,3-dimethylvalerate.
[Process 6] 4M aqueous solution of lithium hydroxide → 1M aqueous solution of sodium hydroxide.
LC-MS: the calculated value=432.15, and the measured value $(M+1)^+$=433.2

[Example 10]

Production of 4-(1-((1-naphthyl)methyl)-5-trifluoromethylbenzimidazole-2-ylthio)butyric acid (Compound No. 126)

4-(1-((1-naphthyl)methyl)-5-trifluoromethylbenzimidazole-2-ylthio)-butyric acid was obtained by employing the same procedure described in Example 3, but changing the reagents partially as follows.
[Process 1] 4-cyano-2-nitroaniline → 4-trifluoromethyl-2-nitroaniline.
[Process 5] potassium carbonate → triethyl amine.
[Process 6] 4M aqueous solution of lithium hydroxide → 1M aqueous solution of sodium hydroxide
LC-MS: the calculated value=444.11, and the measured value $(M+1)^+$=445.3

[Example 11]

Production of 4-(1-((2,5-dimethylphenyl)methyl)-5-methoxybenzimidazole-2-ylthio)butyric acid (Compound No. 495)

4-(1-((2,5-dimethylphenyl)methyl)-5-methoxybenzimidazole-2-ylthio)but yric acid was obtained by employing

the same procedure described in Example 3, but changing the reagents partially as follows.
[Process 1] 4-cyano-2-nitroaniline → 4-methoxy-2-nitroaniline
[Process 2] sodium hydroxide→potassium carbonate;
1-bromomethylnaphthalene → 2,5-dimethyl-1-chloromethylbenzene
[Process 6] 4M aqueous solution of lithium hydroxide → 2M aqueous solution of sodium hydroxide.
LC-MS: the calculated value=384.15, and the measured value $(M+1)^+$=385.3

[Example 12]

Production of 4-(1-((2,5-dimethylphenyl)methyl)-5-cyanobenzimidazole-2-ylthio)butyric acid (Compound No. 337)

4-(1-((2,5-dimethylphenyl)methyl)-5-cyanobenzimidazole-2-ylthio)-butyric acid was obtained by employing the same procedure described in Example 3, but changing the reagents partially as follows.
[Process 2] sodium hydroxide→potassium carbonate;
1-bromomethylnaphthalene → 2,5-dimethyl-1-bromomethylbenzene
[Process 6] 4M aqueous solution of lithium hydroxide → 2M aqueous solution of sodium hydroxide.
LC-MS: the calculated value=379.14, and the measured value $(M+1)^+$=380.3.

[Example 13]

Production of 4-(1-((1-naphthyl)methyl)-5-ethoxybenzimidazole-2-ylthio)butyric acid (Compound No. 128)

4-(1-((1-naphthyl)methyl)-5-ethoxybenzimidazole-2-ylthio)butyric acid was obtained by employing the same procedure described in Example 1, but
changing the reagents partially as follows.
[Process 1] 4-methoxy-2-nitroaniline → 4-ethoxy-2-nitroaniline
LC-MS: the calculated value=420.15, and the measured value $(M+1)^+$=421.3.

[Example 14]

Production of 3-(1-((1-naphthyl)methyl)-benzimidazole-2-ylthio)propanoic acid (Compound No. 496)

3-(1-((1-naphthyl)methyl)-benzimidazole-2-ylthio)propanoic acid was obtained by employing the same procedure described in Example 3, but changing the reagents partially as follows.
[Process 1] 4-cyano-2-nitroaniline → 2-nitroaniline
[Process 5] ethyl 4-bromo-n-butyrate→ ethyl 3-bromo-propanoate
LC-MS: the calculated value=362.11, and the measured value $(M+1)^+$=363.3.

[Example 15]

Production of 5-(1-((1-naphthyl)methyl)-benzimidazole-2-ylthio)valeric acid (Compound No. 497)

5-(1-((1-naphthyl)methyl)-benzimidazole-2-ylthio)valeric acid was obtained by employing the same procedure described in Example 3, but changing the reagents partially as follows.
[Process 1] 4-cyano-2-nitroaniline → 2-nitroaniline
[Process 5] ethyl 4-bromo-n-butyrate→ ethyl 5-bromovalerate
LC-MS: the calculated value=390.14, and the measured value $(M+1)^+$=391.3.

[Example 16]

Production of 4(1-((1-naphthyl)methyl)-5,6-dimethylbenzimidazole-2-ylthio)butyric acid (Compound No. 129)

4-(1-((1-naphthyl)methyl)-5,6-dimethylbenzimidazole-2-ylthio)butyric acid was obtained by employing the same procedure described in Example 1; however, the reactions were carried out from Process 4 by using a commercially available 2-amino-4,5-dimethylaniline.
LC-MS: the calculated value=404.16, and the measured value $(M+1)^+$=405.3.

[Example 17]

Production of 4(1-((1-naphthyl)methyl)-5,6-dimethylbenzimidazole-2-ylsulfonyl)butyric acid (Compound No. 498)

83 mg of ethyl 4-(1-((1-naphthyl)methyl)-5,6-dimethylbenzimidazole-2-ylthio)butyrate (0.19 mmol) obtained in Process 5 of Example 16 was dissolved in 5 ml of methylene chloride, to the solution was added 70 mg of MCPBA (0.40 mmol), and the resulting mixture was stirred at room temperature for 4 hr. After the finish of the reaction, the reaction mixture was extracted with chloroform after the addition of a saturated aqueous solution of sodium hydrogencarbonate. The organic layer was dried over magnesium sulfate, and the solvent was removed in vacuo. The obtained residue was dissolved in 5 ml of methanol, to the solution was added 1 ml of a 4M aqueous solution of lithium hydroxide, and the mixture was stirred at room temperature for 4 hr. After adding 6M hydrochloric acid, the reaction mixture was extracted with chloroform, the organic layer was dried over magnesium sulfate, and the solvent was removed in vacuo. The obtained residue was purified by silica gel chromatography (chloroform : methanol = 20:1) to yield 50.2 mg of 4-(1-((1-naphthyl)methyl)-5,6-dimethylbenzimidazole-2-ylsulfonyl)butyric acid (0.115 mmol, 61% yield in two steps).
LC-MS: the calculated value=436.15, and the measured, value $(M+1)^+$=437.2.

[Example 18]

Production of 4-(1-((2,5-dimethylphenyl)methyl)-7-azabenzimidazole-2-ylthio)butyric acid

4(1-((2,5-dimethylphenyl)methyl)-7-azabenzimidazol-2-ylthio)butyric acid was obtained by employing the same procedure described in Example 1, but changing the reagents partially as follows.
[Process 1] 4-methoxy-2-nitroaniline → 2-amino-3-nitropyridine.
LC-MS: the calculated value=355.14, and the measured value $(M+1)^+$=356.2

[Example 19]

Preparation of recombinant human mast cell chymase

A recombinant pro-type human mast cell chymase was prepared according to the report by Urata et al., Journal of Biological Chemistry 17173, Vol. 266 (1991). That is, the product was purified from the supernatant of the culture broth of insect cell (Tn 5) infected with a recombinant baculovirus containing a cDNA encoding a human mast cell chymase by heparin sepharose (manufactured by Pharmacia Co.). The obtained chymase was further activated according to the report of Murakami et al., Journal of Biological Chemistry, 2218, Vol. 270 (1995), and then it was purified by heparin sepharose to yield an active-type human mast cell chymase.

[Example 20]

Measuring the inhibition of enzyme activity of recombinant human mast cell chymase

To 50 µl of buffer A (0.5 to 3.0 M NaCl, 50 mM tris-HCl, pH 8.0) containing 1 to 5 ng of the active-type human mast cell chymase obtained by Example 19 was added 2 µl of a DMSO solution containing a compound of the present invention, and the mixture was made to react at room temperature for 5 min after the addition of 50 µl of the buffer A containing 0.5 mM of succinyl-alanyl-histidyl-prolyl-phenylalanylparanitroanilide (Bacchem Co.) as a substrate. The inhibition activity was determined by measuring the variation of the absorption at 405 nM with the passage of time.
As a result, an inhibition activity of $IC_{50}$=1 to 10 nM was observed in each of Compounds No. 121, 127, 129, 492 and 493. An inhibition activity of $IC_{50}$=10 to 100 nM was observed in each of Compounds No. 126, 128, 131, 337, 363, 494, 495, 496, 497 and 498.
As shown above, a benzimidazole derivative of the present invention exhibits a strong chymase inhibition activity. It has become clear that the compound is therefore clinically applicable as an inhibitor against human chymase activity which is useful for the prevention and/or treatment of various kinds of diseases associated with a human chymase.

[Example 21]

Measuring blood concentration in forced oral administration into rat stomach

By using a SD male rat, a compound having the compound number of 121, 127 or 363 was forcedly administered at a dose of 30 mg/kg into the stomach under starvation, and blood samples were taken out at 30 min, 1 hr, 2 hr, 4 hr and 8 hr after the administration. Directly after the collection of the blood samples, the serum component was separated, the compound of the present invention was extracted by a general method of solid extraction, and the obtained sample was subjected to HPLC analysis by using an ODS column to measured the amount of the unchanged compound. The results are shown in the following table.

| Compound No. | AUC (extrapolation) (μg×hr/mL) | Cmax (μg/mL) |
|---|---|---|
| 121 | 226 | 122 |
| 127 | 181 | 76 |
| 363 | 182 | 77 |

It has become clear from the result shown above that the compounds of the present invention belong to a pharmaco-kinetically excellent group. Especially, the group of the compounds whose B is $-CH_2CH_2CH_2-$ is pharmaco-kinetically excellent.

[Example 22]

In vitro metabolic test using hepatic microsome (Ms)

Measuring method

○ Reaction solution composition and reaction conditions

| | | Composition and Operation | | Note |
|---|---|---|---|---|
| A | Composition | Name of Reagent | Final Concentration | Amount of Reaction Solution<br><br>0.5 mL |
| | Buffer | phosphate buffer (pH 7.4) | 0.1 M | |
| | Chelating Agent | EDTA | 1.0 mM | |
| | NADPH Generation System | magnesium chloride | 3.0 mM | |
| | | G6P | 5.0 mM | |
| | | G6PDH | 1.0 IU | |
| | Enzyme | hepatic microsome | 1.0 mg/mL | |
| | Substrate | substrate (test compound) | 5.0 μM | |
| | Reaction Starting Liquid | NADPH | 1.0 mM | |
| Reaction Conditions | | 37°C incubation (water bath, vibration)<br>reaction time: 0, 2, 5, 10 and 30 min | | |
| Reaction Stopping Liquid (Extraction Liquid) | | acetonitrile | | 3 times the amount of reaction liquid |
| Protein Removal | | 3000 rpm, 10 min centrifugation<br>collect supernatant, and solvent removal by rotary evaporator | | |
| Liquid of Redissolution | | redissolution in moving phase liquid of analytical HPLC | | |
| Analysis | | detect the peak of unchanged body in HPLC by UV detector | | |

A: composition of reconstruction system

○ Method for calculating MR

Letting the amount of the unchanged body at the initial concentration (reaction time 0 min) 100%, the speed of metabolism is determined from the amount of the decrease of the unchanged body at each reaction time, and the maximum speed of metabolism (MR) is evaluated.

MR=(the substrate concentration at reaction time 0 min - the substance

concentration after the reaction) ÷ reaction time ÷ protein concentration

(nmol/min/mg-protein)

Following measurement results were obtained by this method.

| Compound No. | MR | Remaining Rate (%) of Substrate at 30 min |
|---|---|---|
| 121 | 0.235 | 56.6 |
| 127 | 0.190 | 21.6 |
| 363 | 0.147 | 57.6 |

From the above results, it has become clear that compounds of the present invention belong to a group which is stable in metabolism. Especially, the group of the compounds having B of $-CH_2CH_2CH_2-$ is stable in metabolism.

## Industrial Field of Application

Benzimidazole derivatives or their salts, which are active ingredients of inhibitors against human chymase activity of the present invention, exhibit strong human chymase inhibiting activity. Accordingly, the inhibiting agent against human chymase activity of the present invention can be clinically applicable as a preventing agent and/or a treating agent for various diseases associated with human chymase.

## Claims

**1.** An inhibitor against human chymase activity containing a benzimidazole derivative expressed by the following formula (1) or its salt as an active ingredient,

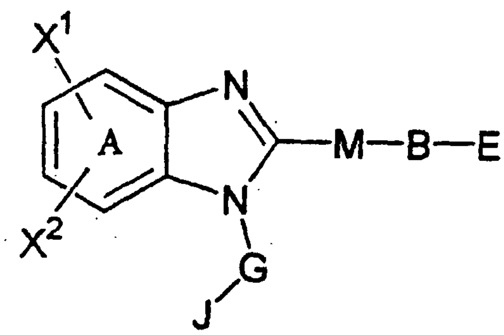

(1)

[in the formula (1), the ring marked with A expresses a pyridine ring or a benzene ring;

$X^1$ and $X^2$ are each at the same time or independently a hydrogen atom, a halogen atom, a trihalomethyl group, a hydroxyl group, a nitro group, a cyano group, $-CH_2NH_2$, $-CH=NR^1$, $-CH=NOR^1$ or $-CONR^1R^2$ (here, $R^1$ and $R^2$ are each a hydrogen atom or a $C_{1-4}$ alkyl group), $-COOR^3$ (here, $R^3$ is a hydrogen atom or a $C_{1-4}$ alkyl group), a substituted or unsubstituted $C_{1-6}$ normal, cyclic or branched alkyl group, a substituted or unsubstituted $C_{3-7}$ cycloalkyl group, a substituted or unsubstituted $C_{1-6}$ normal or branched alkoxyl group, a

substituted or unsubstituted $C_{1-6}$ normal or branched alkylthio group, a substituted or unsubstituted $C_{1-6}$ normal or branched alkylsulfonyl group or a substituted or unsubstituted $C_{1-6}$ normal or branched alkylsulfinyl group {the substituent permissible to the groups is a halogen atom, a hydroxyl group, a nitro group, a cyano group, an acyl group, a trihalomethyl group, a trihalomethoxy group, a phenyl group, an oxo group or a phenoxy group optionally substituted with one or more halogen atoms, and the substituent may substitute singly or plurally independently at arbitrary position(s)};

B is a substituted or unsubstituted $C_{1-6}$ normal, cyclic or branched alkylene group or a substituted or unsubstituted $C_{2-6}$ normal or branched alkenylene group {the substituent permissible to the groups is a halogen atom, a hydroxyl group, a nitro group, a cyano group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched acyl group, a $C_{1-6}$ normal or branched acylamino group, a trihalomethyl group, a trihalomethoxy group, a phenyl group, an oxo group or a phenoxy group optionally substituted with one or more halogen atoms, and the substituent may substitute singly or plurally independently at arbitrary position(s) of the alkylene group or an alkenylene group; between atoms, the alkylene group or alkenylene group optionally contains one or more of -O-, -S-, $-SO_2-$ or $-NR^4-$, but this atom or atomic group does not bond directly to the M, and here $R^4$ is a hydrogen atom or a $C_{1-6}$ normal or branched alkyl group};

E expresses $-COOR^4$, $-SO_3R^4$, $-CONHR^5$, $-SO_2NHR^4$, $-PC(OR^6)_2$, a tetrazol-5-yl group, a 5-oxo-1,2,4-oxadiazol-3-yl group or a 5-oxo-1,2,4-thiadiazol-3-yl group (here, $R^4$ is similarly defined as above; $R^5$ is a hydrogen atom, a cyano group, or a $C_{1-6}$ normal or branched alkyl group; $R^6$ is a hydrogen atom, a $C_{1-6}$ normal or branched alkyl group, or trifluoromethylsulfonyl group, or its pharmaceutically permissible salt);

G is a substituted or unsubstituted $C_{1-6}$ normal or branched alkylene group {between atoms, the alkylene group optionally contains one or more of -O-, -S-, $-SO_2-$ or $-NR^4-$, but this atom or atomic group does not bond directly to the nitrogen atom of the imidazole ring ($R^4$ is similarly defined as above), and the substituent is a halogen atom, a hydroxyl group, a nitro group, a cyano group, a $C_{1-6}$ normal or branched alkoxyl group (including the case where adjacent two groups form an acetal bonding), trihalomethyl group, a trihalomethoxy group, a phenyl group or an oxo group};

J is a substituted or unsubstituted $C_{1-6}$ normal, cyclic or branched alkyl group, a substituted or unsubstituted $C_{4-10}$ aryl group {the substituent. permissible to the groups is a halogen atom, a hydroxyl group, a nitro group, a cyano group, $-COOR^7$ (here, $R^7$ is a hydrogen atom or a $C_{1-4}$ alkyl group), a $C_{1-6}$ normal, cyclic or branched alkyl group, a $C_{1-6}$ normal or branched alkoxyl group (including the case where adjacent two groups form an acetal bonding), a $C_{1-6}$ normal or branched alkylthio group, a $C_{1-6}$ normal or branched alkylsulfonyl group, a $C_{1-6}$ normal or branched alkylsulfinyl group, a $C_{1-6}$ acyl group, a $C_{1-6}$ normal or branched acylamino group, a trihalomethyl group, a trihalomethoxy group, a phenyl group, an oxo group, or a phenoxy group optionally substituted with one or more halogen atoms; the substituent may substitute singly or plurally independently at arbitrary position(s) of the alkyl group or aryl group; and the substituent is further optionally substituted with a halogen atom, a hydroxyl group, a nitro group, a cyano group, an acyl group, a trihalomethyl group, a phenyl group, an oxo group or a phenoxy group optionally substituted with a halogen atom}; and

M is a sulfur atom, a sulfinyl group, a sulfonyl group, a single bond or $-CR^8R^{9-}$ (here, $R^8$ and $R^9$ are each at the same time or independently a hydrogen atom or a $C_{1-4}$ alkyl group)].

**2.** An inhibitor against human chymase activity set forth in Claim 1 wherein the ring marked with A in the above formula (1) is a benzene ring.

**3.** An inhibitor against human chymase activity set forth in Claim 1 wherein the ring marked with A in the above formula (1) is a pyridine ring.

**4.** An inhibitor against human chymase activity set forth in one out of Claims 1 to 3 wherein $X^1$ and $X^2$ in the above formula (1) are each at the same time or independently a hydrogen atom, a halogen atom, a trihalomethyl group, a cyano group, a substituted or unsubstituted $C_{1-3}$ normal or branched alkyl group, a substituted or unsubstituted $C_{1-3}$ normal or branched alkoxyl group, or a substituted or unsubstituted $C_{1-3}$ normal or branched alkylthio group.

**5.** An inhibitor against human chymase activity set forth in one out of Claims 1 to 4 wherein J in the above formula (1) is a group described in the following formula (2) or (3),

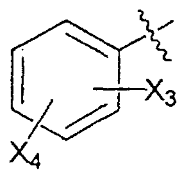 (2) 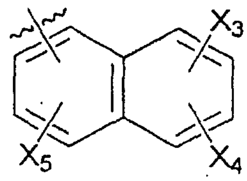 (3)

[here, $X^3$, $X^4$ and $X^5$ are each at the same time or independently a hydrogen atom, a halogen atom, a hydroxyl group, a nitro group, a cyano group, a trihalomethyl group, a trihalomethoxy group, $-COOR^7$ (here, $R^7$ is a hydrogen atom or a $C_{1-4}$ alkyl group), a substituted or unsubstituted $C_{1-3}$ normal or branched alkyl group, a substituted or unsubstituted $C_{1-3}$ normal or branched alkoxyl group, a substituted or unsubstituted $C_{1-3}$ normal or branched alkylthio group, a substituted or unsubstituted $C_{1-3}$ normal or branched alkylsulfonyl group, or a substituted or unsubstituted $C_{1-3}$ normal or branched alkylsulfinyl group; there is no limitation regarding the substitution positions of $X^3$, $X^4$ and $X^5$ on the benzene ring or the naphthalene ring].

**6.** An inhibitor against human chymase activity set forth in one out of Claims 1 to 5 wherein M in the above-mentioned formula (1) is a sulfur atom.

**7.** An inhibitor against human chymase activity set forth in one out of Claims 1 to 6 wherein B in the above-mentioned formula (1) is a substituted or unsubstituted $C_{1-6}$ normal, cyclic or branched alkylene group.

**8.** An inhibitor against human chymase activity set forth in one out of Claims 1 to 7 wherein G in the above-mentioned formula (1) is $-CH_2-$, $-CH_2CH_2-$, $-CH_2CO-$, $-CH_2CH_2O-$, $-CH_2CONH-$, $-CO-$, $-SO_2-$, $-CH_2SO_2-$, $-CH_2S-$ or $-CH_2CH_2S-$(J bonds to the right side of said group).

**9.** An inhibitor against human chymase activity set forth in one out of Claims 1 to 8 wherein E in the above-mentioned formula (1) is -COOH.

**10.** A benzimidazole derivative expressed by the following formula (4) or its pharmaceutically permissible salt,

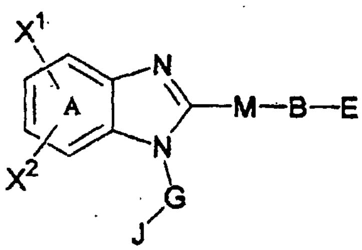 (4)

[in the formula (4), the definitions of the ring marked with A, and $X^1$, $X^2$, B, E, G, J and M are same as those in the above formula (1); however, excepting the case where at least one of $X^1$ and $X^2$ is a cyano group, $-CH_2NH_2$, $-CH=NR^1$, $-CH=NOR^1$ or $-CONR^1R^2$ (here, $R^1$ and $R^2$ are each a hydrogen atom or a $C_{1-4}$ alkyl group), J expresses only a substituted naphthalene ring].

**11.** A benzimidazole derivative or its pharmaceutically permissible salt set forth in Claim 10 wherein $X^1$ and $X^2$ in the above formula (4) are each a hydrogen atom, a cyano group, $-CH_2NH_2$, $-CH=NR^1$, $-CH=NOR^1$ or $-CONR^1R^2$ (here, $R^1$ and $R^2$ are each a hydrogen atom or a $C_{1-4}$ alkyl group; $X^1$ and X2 are not hydrogen at the same time).

**12.** A benzimidazole derivative or its pharmaceutically permissible salt set forth in Claim 10 wherein $X^1$ and $X^2$ in the above formula (4) are each at the same time or independently a hydrogen atom, a halogen atom, a trihalomethyl group, a hydroxyl group, a nitro group, $-CH=NR^1$ (here, $R^1$ is a hydrogen atom or a $C_{1-4}$ alkyl group), $-COOR^3$ (here, $R^3$ is a hydrogen atom or a $C_{1-4}$ alkyl group), a substituted or unsubstituted $C_{1-6}$ normal, cyclic or branched alkyl group, a substituted or unsubstituted $C_{3-7}$ cycloalkyl, a substituted or unsubstituted $C_{1-6}$ normal or branched alkoxyl group, a substituted or unsubstituted $C_{1-6}$ normal or branched alkylthio group, a substituted or unsubstituted $C_{1-6}$ normal or branched alkylsulfonyl group or a substituted or unsubstituted $C_{1-6}$ normal or branched alkylsulfinyl group {the substituent permissible to the groups is a halogen atom, a hydroxyl group, a nitro group, a cyano group,

an acyl group, a trihalomethyl group, a trihalomethoxy group, a phenyl group, an oxo group or a phenoxy group optionally substituted with one or more halogen atoms, and the substituent may substitute singly or plurally independently at arbitrary position(s)}.

**13.** A benzimidazole derivative or its pharmaceutically permissible salt set forth in Claim 10 wherein $X^1$ and $X^2$ in the above formula (4) are each a hydrogen atom or a cyano group (here, $X^1$ and $X^2$ can not be hydrogen toms at the same time).

**14.** A benzimidazole derivative or its pharmaceutically permissible salt set forth in one out of Claims 10 to 13 wherein M in the above formula (4) is a sulfur atom.

**15.** A benzimidazole derivative or its pharmaceutically permissible salt set forth in one out of Claims 10 to 14 wherein B in the above formula (4) is a substituted or unsubstituted $C_{1-6}$ normal, cyclic or branched alkylene group.

**16.** A benzimidazole derivative or its pharmaceutically permissible salt set forth in one out of Claims 10 to 15 wherein J in the above formula (4) is a group expressed by the following formula (2) or (3),

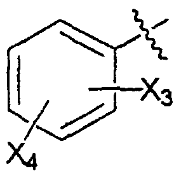 (2) 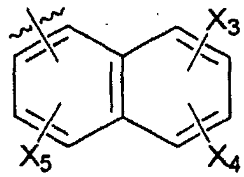 (3)

[here, $X^3$, $X^4$ and $X^5$ are each at the same time or independently a hydrogen atom, a halogen atom, a hydroxyl group, a nitro group, a cyano group, a trihalomethyl group, a trihalomethoxy group, $-COOR^7$ (here, $R^7$ is a hydrogen atom or a $C_{1-4}$ alkyl group), a substituted or unsubstituted $C_{1-3}$ normal or branched alkyl group, a substituted or unsubstituted $C_{1-3}$ normal or branched alkoxyl group, a substituted or unsubstituted $C_{1-3}$ normal or branched alkylthio group, a substituted or unsubstituted $C_{1-3}$ normal or branched alkylsulfonyl group, or a substituted or unsubstituted $C_{1-3}$ normal or branched alkylsulfinyl group; there is no limitation regarding the substitution positions of $X^3$, $X^4$ and $X^5$ on the benzene ring or the naphthalene ring].

**17.** A benzimidazole derivative or its pharmaceutically permissible salt set forth in one out of Claims 10 to 16 wherein G in the above formula (4) is $-CH_2-$, $-CH_2CH_2-$, $-CH_2CO-$, $-CH_2CH_2O-$, $-CH_2CONH-$, $-CO-$, $-SO_2-$, $-CH_2SO_2-$, $-CH_2S-$ or $-CH_2CH_2S-$ (J bonds to the right side of said group).

**18.** A benzimidazole derivative or its pharmaceutically permissible salt set forth in one out of Claims 10 to 17 wherein E in the above formula (4) is COOH.

**19.** A pharmaceutical composition consisting of a benzimidazole derivative and/or its pharmaceutically permissible salt set forth in one out of Claims 10 to 18, and a pharmaceutically permissible carrier.

**20.** A chymase activity inhibitor set forth in one out of Claims 1 to 9 whose targeting disease is an inflammatory disease, an allergy disease, a respiratory disease, a cardiovascular disease or a bone/cartridge metabolic disease.

**21.** A human chymase activity inhibitor set forth in Claim 20 which is a preventing agent or a treating agent of a disease.

# INTERNATIONAL SEARCH REPORT

International application No.
PCT/JP01/00272

A. CLASSIFICATION OF SUBJECT MATTER
Int.Cl⁷ C07D235/28, C07D235/16, C07D471/04, A61K31/4184, A61K31/437, A61P43/00, A61P29/00, A61P37/08, A61P11/00, A61P9/00, A61P3/14

According to International Patent Classification (IPC) or to both national classification and IPC

B. FIELDS SEARCHED

Minimum documentation searched (classification system followed by classification symbols)
Int.Cl⁷ C07D235/28, C07D235/16, C07D471/04, A61K31/4184, A61K31/437, A61P43/00, A61P29/00, A61P37/08, A61P11/00, A61P9/00, A61P3/14

Documentation searched other than minimum documentation to the extent that such documents are included in the fields searched

Electronic data base consulted during the international search (name of data base and, where practicable, search terms used)
CAPLUS(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| PA | WO, 00/03997, A (Teijin Limited), 27 January, 2000 (27.01.00) (Family: none) | 1-21 |
| X | US, 5124336, A (Laboratoires UPSA), 23 June, 1992 (23.06.92) | 1-9,20,21 |
| A | & FR, 2658511, A | 10-19 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 April, 2001 (12.04.01) | 01 May, 2001 (01.05.01) |

| Name and mailing address of the ISA/ | Authorized officer |
|---|---|
| Japanese Patent Office | |
| Facsimile No. | Telephone No. |

Form PCT/ISA/210 (second sheet) (July 1992)